(19) Europäisches Patentamt
European Patent Office
Office européen des brevets

(11) Numéro de publication : **0 360 644 B1**

(12) **FASCICULE DE BREVET EUROPEEN**

(45) Date de publication du fascicule du brevet : **17.02.93 Bulletin 93/07**

(51) Int. Cl.$^5$ : **A61K 7/13**

(21) Numéro de dépôt : **89402361.3**

(22) Date de dépôt : **30.08.89**

(54) **Composition tinctoriale d'oxydation contenant au moins une base double associée à au moins une base simple et procédé de teinture la mettant en oeuvre.**

(30) Priorité : **08.09.88 FR 8811739**

(43) Date de publication de la demande : **28.03.90 Bulletin 90/13**

(45) Mention de la délivrance du brevet : **17.02.93 Bulletin 93/07**

(84) Etats contractants désignés : **AT BE CH DE ES FR GB GR IT LI NL SE**

(56) Documents cités :
**DE-A- 2 934 329**
**FR-A- 2 016 123**
**GB-A- 2 018 302**
**GB-A- 2 018 836**
**GB-A- 2 205 329**

(73) Titulaire : **L'OREAL**
**14, Rue Royale**
**F-75008 Paris (FR)**

(72) Inventeur : **Madrange, Annie**
**23, rue Diderot**
**F-78100 Saint-Germain-en-Laye (FR)**
Inventeur : **Millequant, Jean-Marie**
**144, rue Garibaldi**
**F-94100 Saint-Maur (FR)**

(74) Mandataire : **Casalonga, Axel et al**
**BUREAU D.A. CASALONGA - JOSSE**
**Morassistrasse 8**
**W-8000 München 5 (DE)**

**EP 0 360 644 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à des compositions destinées à être utilisées dans la teinture des fibres kératiniques, en particulier des fibres kératiniques humaines, et notamment les cheveux humains, contenant des bases d'oxydation dites "doubles" et des bases d'oxydation dites "simples" et aux procédés de teinture mettant en oeuvre de telles compositions.

Il est connu d'utiliser pour la teinture des fibres kératiniques, en particulier des cheveux humains, des compositions tinctoriales contenant des colorants d'oxydation tels que des composés que l'on appelle également "bases d'oxydation ou précurseurs de colorants d'oxydation".

Ces bases d'oxydation ne sont pas des colorants en eux-mêmes, mais sont susceptibles de développer leur pouvoir tinctorial en milieu oxydant pour donner naissance par un processus de condensation oxydative, soit du précurseur de colorant d'oxydation sur lui-même, soit de la base ou précurseur de colorant d'oxydation sur un composé appelé "nuanceur ou coupleur".

Ces précurseurs de colorants d'oxydation peuvent être utilisés seuls ou en association avec d'autres colorants, notamment des colorants "directs", tels que les colorants nitrés de la série benzénique, les anthraquinones et les azoïques.

Les bases "d'oxydation" et plus particulièrement les paraphénylènediamines présentent des avantages indéniables. Elles possèdent une bonne affinité pour les fibres kératiniques sur lesquelles, en général, elles permettent de produire une grande variété de couleurs, résistant de manière satisfaisante aux radiations lumineuses et aux lavages répétés.

Cependant, si avec les précurseurs de colorants d'oxydation appartenant à la famille des paraphénylènediamines, on obtient des résultats tinctoriaux et des ténacités convenables sur la plupart des chevelures, on constate cependant un rejet de la coloration et une mauvaise ténacité, quand ces compositions tinctoriales sont appliquées sur les cheveux ayant subi, de façon répétée, des traitements capillaires sensibilisants, tels que des décolorations ou des permanentes, ou encore des décolorations et des permanentes.

Ainsi, sur des cheveux dont une partie est très sensibilisée, on obtient, dès le jour de la coloration, des écarts importants de nuances entre la partie proche de la racine et la pointe très sensibilisée. On constate également, du fait de la mauvaise tenue sur la partie abîmée, que cet écart va en augmentant avec le temps au lieu de se réduire au fil des jours et des shampooings.

Pour remédier à ce problème, une première solution proposée antérieurement, dans l'état de la technique, a consisté à introduire dans les compositions tinctoriales des macromolécules cationiques qui, en réduisant la sensibilisation, permettent un meilleur unisson et conduisent à des nuances résistant déjà mieux au lavage.

Une seconde solution a été l'introduction dans les compositions tinctoriales de bases et/ou de coupleurs présentant un poids moléculaire élevé, obtenus à l'aide de précurseurs de colorants d'oxydation portant des substitutions diverses sur les atomes d'azote ou directement sur le noyau benzénique. Cette solution permet de conduire à des nuances plus unies et plus tenaces quand une partie de la chevelure est moyennement sensibilisée et surtout lorsqu'on considère les nuances sombres qui vont du châtain au noir.

La demanderesse a cependant constaté que même pour des formulations ainsi améliorées, les performances restent encore relativement modestes quand la pointe des cheveux est très sensibilisée et quand on souhaite obtenir sur l'ensemble de la chevelure des nuances unies et tenaces allant du châtain au blond clair. On constate, en effet, un manque d'unisson par rejet de la coloration et une mauvaise tenue aux lavages répétés.

La demanderesse a découvert, ce qui fait l'objet de l'invention, qu'il était possible d'obtenir des gains importants d'unisson et de tenue aux lavages répétés, en associant suivant les règles qui seront définies ci-après, à des bases d'oxydation "simples" de la famille des paraphénylènediamines, des bases d'oxydation plus complexes de la famille des N,N′-diphénylalkylènediamines, encore appelées "bases doubles".

Ces bases sont pour la plupart connues en elles-mêmes et sont décrites, notamment, dans le brevet français 2.016.123.

La demanderesse a encore découvert, de façon surprenante, qu'il était possible d'obtenir sur toute la longueur des cheveux, même fortement sensibilisés sur une partie de ceux-ci, une coloration présentant un bon unisson et conservant cette propriété, même après des lavages répétés.

L'invention a donc pour objet des compositions tinctoriales pour fibres kératiniques et en particulier pour cheveux humains, comprenant l'association définie ci-dessus.

Un autre objet de l'invention est constitué par le procédé de teinture des fibres kératiniques, en particulier des cheveux humains mettant en oeuvre une telle association.

D'autres objets de l'invention apparaîtront à la lecture de la description et des exemples qui suivent.

La composition tinctoriale conforme à l'invention est essentiellement caractérisée par le fait qu'elle comprend dans un milieu approprié pour la teinture des fibres kératiniques :

(a) au moins un précurseur de colorant appartenant à la famille (A) des bases simples choisies parmi les paraphénylènediamines de formule :

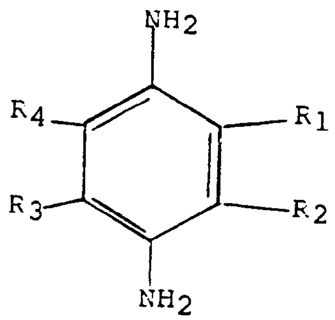

(I)

dans laquelle $R_1$, $R_2$, $R_3$, $R_4$, indépendamment l'un de l'autre, représentent un atome d'hydrogène, d'halogène, un radical alkyle ou alcoxy, ainsi que les sels de ces composés;

(b) au moins un précurseur de colorant de la famille (B) des bases doubles choisies parmi les N,N'-diphénylalcoylènediamines, répondant à la formule :

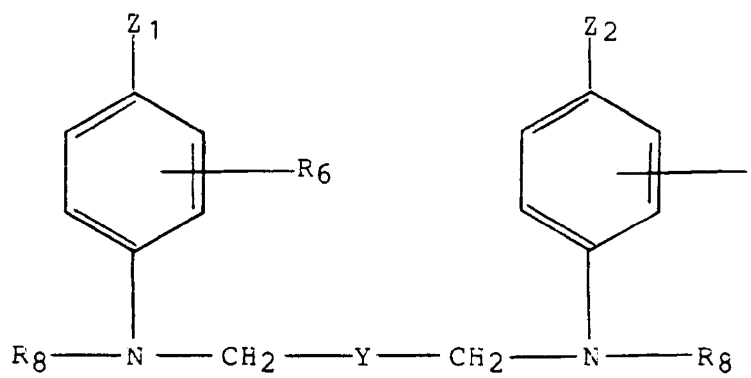

(II)

dans laquelle :

$Z_1$ et $Z_2$, identiques ou différents, représentent des groupements hydroxyle ou $NHR_9$, dans lequel $R_9$ désigne un atome d'hydrogène ou un radical alkyle;

$R_6$ et $R_7$, identiques ou différents, représentent, soit des atomes d'hydrogène, soit des atomes d'halogène, soit encore des groupements alkyle;

$R_8$ représente un atome d'hydrogène, un groupement alkyle, hydroxyalkyle ou aminoalkyle, le reste amino peut être substitué par un groupement alkyle;

Y représente un radical pris dans le groupe constitué par les radicaux suivants :

$$-(CH_2)_n-,\ -(CH_2)_{n'}-O-(CH_2)_{n'}-,$$
$$-(CH_2)_{n'}-CHOH-(CH_2)_{n'},\ -(CH_2)_{n'}-N(CH_3)-(CH_2)_{n'}-;$$

n étant un nombre entier compris entre 0 et 8 et n' étant un nombre entier compris entre 0 et 4, ainsi que leurs sels d'addition avec des acides.

Dans les formules (I) et (II), les groupements alkyle et alcoxy ont de préférence de 1 à 4 atomes de carbone, le groupement hydroxyalkyle a de préférence de 2 à 4 atomes de carbone et le groupement aminoalkyle a de préférence de 1 à 4 atomes de carbone.

Les sels sont choisis parmi les sels cosmétiquement acceptables, comme les chlorhydrates, les bromhydrates, les sulfates et les sels similaires.

Les colorants des familles (A) et (B) sont choisis de façon à ce que l'intensité de la coloration sur cheveux décolorés teints ($V_D$)et l'intensité sur cheveux décolorés permanentés teints ($V_{DP}$), soit telle que $V_D$-$V_{DP}$=0±0,5, les valeurs d'intensité ou "value" étant déterminées selon la notation MUNSELL.

En ce qui concerne la notation MUNSELL, on pourra se référer aux normes ASTM D1535-68. Selon cette

notation, une couleur est définie par la formule HV/C, dans laquelle les trois paramètres désignent respectivement la nuance (ou hue) (H), l'intensité (ou Value) (V) et la pureté (ou chromaticité) (C), la barre oblique étant une simple convention.

Conformément à l'invention et pour la détermination de la condition reliant les intensités, on appelle "cheveux décolorés", des cheveux qui ont été traités pendant 45 minutes environ avec une solution oxydante de composition suivante :

| | |
|---|---|
| - eau oxygénée à 200 volumes | 8,3 à 25 ml |
| - persulfate de sodium | 25 à 75 g |
| - ammoniaque (d=0,92) | 8,3 à 25 ml |
| - Trilon B (acide éthylènediamine tétracétique) | 0,5 g |
| - eau qs | 300 ml |

Les cheveux sont rincés abondamment, puis lavés avec un shampooing neutralisant ayant la composition suivante :

| | |
|---|---|
| - solution à 180 g/l de laurylsulfate d'ammonium | 25 ml |
| - décahydrate de sulfate de sodium | 1,75 g |
| - solution à 500 g/l de thiosulfate de sodium | 1,5 ml |
| - acide tartrique | 0,3 g |
| - eau qs | 300 ml |

Après lavage, les cheveux sont rincés, puis séchés.

Les quantités d'eau oxygénée, de persulfalte de sodium et d'ammoniaque varient selon le degré (faible, moyen ou fort) de décoloration que l'on souhaite obtenir.

On appelle "cheveux décolorés permanentés", les cheveux qui ont d'abord été décolorés selon la méthode décrite ci-dessus et soumis ensuite à une permanente en appliquant pendant 15 minutes une composition réductrice contenant 8 g d'acide thioglycolique, 2 g d'acide thiolactique, dans 100 g d'eau et ajustée à pH=8,2 par l'ammoniaque.

Après rinçage, on applique pendant 15 minutes une solution "fixatrice" constituée par de l'eau oxygénée à 2,5%. Les cheveux sont rincés, puis séchés.

Il va de soi que la composition conforme à l'invention peut être appliquée sur toute chevelure, sous réserve qu'elle remplisse les conditions définies ci-dessus, lorsqu'elle est appliquée sur cheveux décolorés ou décolorés permanentés.

Les paraphénylènediamines plus partirulièrement utilisables conformément à l'invention, sont choisies parmi la paraphénylènediamine, la paratoluylènediamine, la 2,6-diméthylparaphénylènediamine, la 2,5-diméthylparaphénylènediamine, la 2,3-diméthylparaphénylènediamine, la 2-chloroparaphénylènediamine, la 2-méthoxyparaphénylènediamine, la 2-α-hydroxyméthylparaphénylènediamine, la 2-β-hydroxyéthyl paraphénylènediamine, la 2-isopropylparaphénylènediamine, la 2,6-diméthyl 3-méthoxyparaphénylènediamine.

Le précurseur de colorant d'oxydation préféré appartenant à la famille (A) des bases simples est la paraphénylènediamine.

Les composés de formule (II) sont choisis plus particulièrement parmi la N,N'-bis-[(4-amino)phényl]tétraméthylènediamine, la N,N'-bis-(β-diéthylaminoéthyl)N,N'-bis-[(4-amino)phényl]tétraméthylènediamine, la N-(4-hydroxy)phényl N'[(4'-amino)phényl]éthylènediamine, la N,N'-bis-(β-hydroxyéthyl)N,N'-bis-(4-aminophényl)éthylènediamine, la N,N'-bis-(4'-aminophényl)1,3-diaminopropane-2-ol, la N,N'-bis-(β-hydroxyéthyl)N,N'-bis-(4'-aminophényl)1,3-diaminopropane-2-ol, la N,N'-bis(éthyl)N,N'-bis(4'-amino 3'-méthylphényl)éthylènediamine. Ces bases doubles sont connues, sauf la N,N'-bis-(β-hydroxyéthyl)N,N'-bis-(4'-aminophényl)1,3-diaminopropane-2-ol et la N,N'-bis(éthyl)N,N'-bis(4'-amino 3'-méthylphényl)éthylènediamine qui sont nouvelles

et dont le procédé de préparation est décrit plus loin.

Conformément à l'invention, les colorants de la famille (A) et de la famille (B) sont présents à une concentration totale, inférieure ou égale à 6%, sur la base du poids total de la composition, cette concentration étant comprise entre 0,01 et 6% et de préférence entre 0,02 et 3%.

Le rapport molaire entre les bases simples de la famille (A) et les bases doubles de la famille (B), est compris entre 3 et 10, et en particulier entre 3,5 et 7. La composition ne contient pas d'ions iodures, excepté ceux présents naturellement dans l'eau.

Le brevet GB-A-2.018.836 décrit à l'exemple 18 une composition tinctoriale contenant la N,N′-(para-aminophényl)-tétraméthylènediamine sous forme de tétrachlorhydrate, qui est un précurseur de colorant appartenant à la famille (B) des bases doubles, et la 2,6-diméthylparaphénylènediamine sous forme de dichlorhydrate, qui est un précurseur de colorant appartenant à la famille (A) des bases simples.

Cependant, le rapport molaire entre la base simple et la bas double de cet exemple 18 est en-dehors de l'intervalle compris entre 3 et 10, et en outre la relation $V_D$-$V_{DP}$=±0,5 n'est pas respectée.

GB-A-2.205.329 enseigne un procédé de teinture des fibres kératiniques au moyen d'une composition tinctoriale renfermant des précurseur de colorants d'oxydation en combinaison avec des ions iodures.

GB-A-2.018.302 enseigne un procédé de teinture des fibres kératiniques d'abord avec une composition ayant un pH de 7 à 13 et ensuite, sans rinçage intermédiaire, avec une composition ayant un pH de 1 à 8 et contenant un colorant direct.

DE-A-2.934.329 enseigne une composition tinctoriale renfermant des N,N-bis(4-méthoxy 5-aminophényl)alkylènediamines.

FR-A-2.016.123 enseigne une composition tinctoriale renfermant comme précurseur de colorant d'oxydation une N,N′-diarylalcoylène-α,ω-diamine.

L'unisson de la teinture, c'est-à-dire l'écart de couleur entre les cheveux décolorés teints, d'une part, et les cheveux décolorés, permanentés et teints, d'autre part, est nettement moindre avec la composition tinctoriale selon l'exemple 18 de GB-A-2.018.836 que lorsque, selon la présente invention, le rapport molaire base simple : base double est compris entre 3 et 10 et la relation $V_D$-$V_{DP}$=±0,5 est respecté.

Les autres références concernent des inventions différentes.

Les compositions conformes à l'invention peuvent également contenir, en plus des précurseurs de colorants appartenant aux familles (A) et (B) définies ci-dessus, un ou plusieurs nuanceurs ou coupleurs.

Parmi les coupleurs, on peut citer en particulier les phénols, les métadiphénols, les métaaminophénols, les métaphénylènediamines, les métaacylaminophénols, les méta-uréidophénols, les méta-carbalcoxyaminophénols, l'α-naphtol, les coupleurs possédant un groupe méthylène actif, tels que les composés β-cétoniques et les pyrazolones.

Parmi les phénols, on peut citer le 2-isopropyl 5-méthylphénol.

Parmi les métadiphénols, on peut citer :

la résorcine,
la 2-méthyl résorcine,
la 5-méthyl résorcine,
le 2,4-dihydroxyphénoxyéthanol,
le monoéthyléther de résorcine,
le 2,4-dihydroxy anisole,

Parmi les métaaminophénols, on peut citer :

le métaaminophénol,
le 2-méthyl 5-aminophénol,
le 2-méthyl 5-N-(β-hydroxyéthyl)aminophénol,
le 2-méthyl 5-N(β-mésylaminoéthyl)amino phénol,
le 2,6-diméthyl 3-aminophénol,
la 6-hydroxybenzomorpholine,
et leurs sels.

Parmi les métaphénylènediamines, on peut citer :

la métaphénylènediamine,
le 2,4-diaminophénoxyéthanol,
le 2,4-diméthoxy 1,3-diaminobenzène,
le 1,3,5-triméthoxy 2,4-diaminobenzène,
le 2,4-diamino anisole,
le 6-aminobenzomorpholine,
le [N-2-(β-hydroxyéthyl)amino 4-amino]phénoxy éthanol,
le [N-4-(β-hydroxyéthyl)amino 2-amino]phénoxy éthanol,

le 2-amino N-(4-β-hydroxyéthyl)amino anisole,
le 4,5-di-(β-hydroxyéthoxy)1,3-diaminobenzène,
le 1-β-hydroxyéthoxy 2,4-diaminobenzène,
et leurs sels.

Parmi les autres coupleurs utilisables dans les compositions tinctoriales de l'invention, il faut citer les dérivés du phénol ou de l'aniline comportant un hétérocycle méthylènedioxy et plus particulièrement :
le 3,4-méthylènedioxyphénol,
la 3,4-méthylènedioxyaniline,
le 2-bromo 4,5-méthylènedioxyphénol,
le 2-chloro 4,5-méthylènedioxyphénol,
la 2-méthoxy 4,5-méthylènedioxyaniline,
et les dérivés de pyridine, tels que la 2,6-dihydroxypyridine, la 2,6-diaminopyridine et la 4-méthyl 2,6-dihydroxypyridine.

Les coupleurs, lorsqu'ils sont présents, peuvent être incorporés dans les compositions conformes à l'invention, sous forme libre ou sous forme salifiée, dans des proportions allant jusqu'à 10% et de préférence de 0 à 6% du poids de la composition.

Les compositions conformes à l'invention peuvent également contenir des paraphénylènediamines différentes de celles de formule (I) à fonction amine primaire, en particulier des paraphénylènediamines comportant un groupement amine tertiaire ou secondaire, tel que plus particulièrement le 1-N,N-diméthylamino 4-aminobenzène, le 1-N,N-diéthylamino 4-aminobenzène, le 1-N,N-(bis-β-hydroxyéthyl)amino 4-aminobenzène, le 1-N-β-méthoxyéthylamino 4-aminobenzène, le 1-N-β-hydroxypropyl 4-aminobenzène.

D'autres précurseurs de colorants d'oxydation qui peuvent être présents dans les compositions conformes à l'invention, peuvent être le p-aminophénol et ses dérivés, substitués sur le noyau benzénique ou sur la fonction amine, des dérivés de la pyridine ou de la pyrimidine et plus particulièrement la 2,5-diaminopyridine et ses dérivés N-substitués en position 2, la 2,4,5,6-tétraaminopyrimidine et ses dérivés substitués sur l'atome d'azote.

Les autres précurseurs de colorants peuvent être présents dans les compositions conformes à l'invention dans des proportions pouvant aller jusqu'à 5% en poids par rapport au poids total de la composition.

Les compositions conformes à l'invention peuvent également contenir des colorants directs appartenant plus particulièrement aux familles des colorants benzéniques nitrés, des colorants anthraquinoniques et des colorants azoïques, et ceci en vue d'apporter des reflets à la coloration.

Les colorants directs sont présents dans les compositions conformes à l'invention dans des proportions allant de 0 à 5% et de préférence de 0 à 2% en poids par rapport au poids total de la composition.

Les compositions conformes à l'invention peuvent se présenter sous des formes diverses : liquides plus ou moins épaissis, liquides gélifiables par dilution, crèmes plus ou moins épaisses et peuvent éventuellement être conditionnées en aérosol.

De très nombreux ingrédients cosmétiquement acceptables peuvent concourir à obtenir la forme souhaitée et à réaliser en particulier le produit cosmétique attendu, lorsque la composition est destinée à teindre les cheveux humains vivants.

On peut additionner à ces compositions des solvants cosmétiquement acceptables, des agents tensioactifs, des agents épaississants, des parfums, des agents conservateurs, des agents alcalinisants ou acidifiants, des cires et des corps gras, des agents traitants du cheveu, des agents séquestrants, des agents réducteurs.

Les compositions conformes à l'invention comportent généralement un milieu aqueux constitué par de l'eau ou un mélange eau-solvant(s), le(s) solvant(s) étant choisi(s) préférentiellement parmi les solvants organiques tels que l'alcool éthylique, l'alcool propylique ou isopropylique, l'alcool tertiobutylique, l'alcool benzylique, l'alcool phényléthylique, l'éthylèneglycol, les éthers monométhylique, monoéthylique et monobutylique de l'éthylèneglycol, les polyéthylèneglycols, l'acétate du monoéthyléther de l'éthylèneglycol, le propylèneglycol, les monométhyléthers du propylèneglycol et du dipropylèneglycol, le lactate de méthyle.

Les solvants sont présents dans des concentrations comprises entre 0,5 et 75% en poids par rapport au poids total de la composition, lorsque la composition est constituée par un mélange eau-solvant(s).

Les agents tensio-actifs sont choisis parmi les tensio-actifs anioniques, cationiques, non ioniques, amphotères ou leurs mélanges, et sont présents dans des proportions comprises entre 0,1 et 50% en poids.

Les agents épaississants peuvent être choisis parmi l'alginate de sodium, la gomme arabique, la gomme de guar, les biopolymères comme la gomme de xanthane ou les scléroglucanes, les dérivés de cellulose tels que la méthylcellulose, l'hydroxyéthylcellulose, l'hydroxypropylméthylcellulose, le sel de sodium de la carboxyméthylcellulose et des polymères d'acide acrylique.

On peut également utiliser des agents épaississants minéraux, tels que la bentonite.

Ces épaississants, utilisés seuls ou en mélange, sont présents de préférence dans des proportions comprises entre 0,1 et 5% en poids par rapport au poids total de la composition et avantageusement entre 0,5 et 3% en poids.

Les agents d'acidification utilisables conformément à l'invention, peuvent être choisis parmi l'acide lactique, l'acide acétique, l'acide tartrique, l'acide phosphorique, l'acide chlorhydrique, l'acide citrique.

Les agents alcalinisants sont de préférence choisis parmi les hydroxydes ou carbonates alcalins ou d'ammonium, les amines telles que les alcanolamines, les alkylamines.

Lorsque la composition est utilisée dans un dispositif aérosol, elle peut être conditionnée sous pression en vue de former une mousse en présence d'un agent propulseur et d'au moins un générateur de mousse. Les agents générateurs de mousse peuvent être des polymères moussants, anioniques, cationiques, non ioniques amphotères ou des agents tensio-actif du type de ceux définis ci-dessus.

Les compositions tinctoriales conforme à l'invention sont généralement utilisées en mélange au moment de l'emploi avec un agent oxydant. Cet agent oxydant peut être l'eau oxygénée, le peroxyde d'urée, des persels, tels que les persulfates ou les perborates. Cet agent oxydant est présent dans la composition tinctoriale finale applicable sur les cheveux, dans une proportion allant de 0,5 à 80% en poids.

La composition conforme à l'invention peut être réalisée au préalable et être stockée ou préparée tout juste avant l'emploi, auquel cas, on applique la composition sur les cheveux avec un agent oxydant en utilisant un temps de pose pouvant aller de 5 minutes à 1 heure.

La chevelure, après l'application et la pose, est rincée, éventuellement lavée, puis séchée.

Selon une variante de l'invention, il est possible de stocker les précurseurs de colorants dans des compositions séparées et de procéder, soit au mélange tout juste avant l'emploi, soit appliquer en deux temps ces différentes compositions. On peut appliquer, à ce sujet, dans un premier temps, la composition contenant les précurseurs de colorants de la famille A choisis parmi les paraphénylènediamine, puis après un temps de pose et éventuellement un rinçage, dans un deuxième temps, la deuxième composition contenant les bases doubles.

Les temps de pose pour chacune de ces deux compositions sont compris entre 5 et 45 minutes.

Tout comme précédemment, la chevelure, après le temps de pose, est rincée, lavée éventuellement, rincée à nouveau et séchée.

Dans cette dernière forme de réalisation, la composition conforme à l'invention peut être stockée sous forme d'un kit de teinture, caractérisé par le fait qu'il comprend, dans un premier compartiment, une composition contenant dans un milieu approprié pour la teinture, au moins un précurseur de colorant de la famille (A) des bases simples, et dans un second compartiment, une composition comprenant dans un milieu approprié pour la teinture, au moin un précurseur de colorant de la famille (B) des bases doubles, répondant à la formule (II).

Les compositions présentes dans chacun de ces compartiments pouvant également contenir les autres colorants ou précurseurs de colorants ou ingrédients définis ci-dessus.

Les exemples qui suivent sont destinés à illustrer l'invention.

EXEMPLE DE PREPARATION 1

Préparation de la N,N'-bis-(β-hydroxyéthyl)N,N'-bis-(4-aminophényl)1,3-diaminopropane-2-ol.

1ère étape :

Préparation de la N,N'-bis-(β-hydroxyéthyl)N,N'-bis- (phényl)1,3-diaminopropane-2-ol.

On chauffe 6 heures, au reflux de l'eau, le mélange constitué par 2 moles (274,4 g) de N-β-hydroxy éthylaniline, de 140 g de carbonate de calcium et 1,2 moles (154,8 g) de 1,3-dichloropropane-2-ol dans 1 litre d'eau.

Après refroidissement et neutralisation par 50 ml d'acide chlorhydrique concentré, le produit attendu cristallise.

Après essorage, lavage à l'eau, le produit obtenu est recristallisé de l'alcool isopropylique. On obtient 238 g du produit attendu. Il fond à 120°C.

L'analyse du produit obtenu donne les résultats suivants :

Analyse pour $C_{19}H_{26}N_2O_3$

| | C% | H% | N% | O% |
|---|---|---|---|---|
| Calculé | 69,06 | 7,93 | 8,48 | 14,53 |
| Trouvé | 69,13 | 7,96 | 8,38 | 14,82 |

2ème étape :

Préparation de la N,N'-bis-(β-hydroxyéthyl)N,N'-bis-(4-nitrosophényl)1,3-diaminopropane-2-ol.

A une solution de 0,5 mole (165,2 g) de N,N'-bis-(β-hydroxyéthyl)N,N'-bis-(phényl)1,3-diaminopropane-2-ol préparé à l'étape précédente, de 290 ml d'acide chlorhydrique concentré et de 900 g de glace, on ajoute goutte à goutte entre 0°C et 5°C, une solution de 1,14 moles (78,7 g) de nitrite de sodium dans 150 ml d'eau. L'agitation est maintenue 1 heure 30 après la fin de la coulée. Le milieu réactionnel est neutralisé par ajout de 300 ml d'ammoniaque à 20% à 10°C. Le produit obtenu est, après essorage, réempâté dans l'eau. Il peut être utilisé humide pour l'étape suivante.

3ème étape :

Préparation de l'hydrate du tétrachlorhydrate de N,N'-bis-(β-hydroxyéthyl)N,N'-bis-[(4'-amino)phényl]1,3-diaminopropane-2-ol.

A 930 ml d'alcool éthylique à 96° additionné de 135 ml d'eau, de 11,9 g de chlorure d'ammonium et 390 g de zinc en poudre fine portés au reflux, on ajoute par portions, 0,58 mole (22,6 g) du dérivé dinitroso préparé à l'étape précédente. Le chauffage est maintenu 1 heure à la fin de l'addition.

Par filtration du milieu réactionnel à chaud, on élimine le zinc et précipite le produit attendu par ajout au filtrat, de 340 ml d'une solution d'acide chlorhydrique dans l'éthanol absolu (7,5 N). Par ajout d'éther éthylique, on obtient après séchage, 234 g de produit attendu.

L'analyse du produit obtenu donne les résultats suivants :

Analyse pour $C_{15}H_{26}N_4O_2Cl_4$

| | C% | H% | N% | O% | Cl% |
|---|---|---|---|---|---|
| Calculé | 43,53 | 6,53 | 10,68 | 12,21 | 27,05 |
| Trouvé | 43,72 | 6,57 | 10,37 | 12,16 | 27,10 |

EXEMPLE DE PREPARATION 2

Préparation de l'hydrate du tétrachlorhydrate de N,N'-bis (éthyl) N,N'-bis (4'-amino-3'méthylphényl)éthylène diamine.

1ère étape :

Préparation du dichlorhydrate de N,N'-bis(éthyl)N,N'-bis (3'-méthylphényl)éthylènediamine.

On chauffe 15 heures au reflux le mélange constitué par 1 mole (135,2 g) de N-éthyl m-toluidine, de 60 g de carbonate de calcium et de 0,55 mole (104 g) de dibromo-1,3 éthane.

Après refroidissement et neutralisation par 60 ml d'acide chlorhydrique concentré puis extraction à l'acétate d'éthyle, on obtient le produit attendu qui est transformé en dichlorhydrate par une solution d'acide chlorhydrique dans l'éthanol absolu.

Le produit est recristallisé de l'éthanol absolu.

L'analyse du produit obtenu donne les résultats suivants :

Analyse pour $C_{20}H_{30}N_2Cl_2$

| | C% | H% | N% | Cl% |
|---|---|---|---|---|
| Calculé | 65,04 | 8,18 | 7,58 | 19,20 |
| Trouvé | 65,14 | 8,17 | 7,52 | 19,12 |

2ème étape :

Préparation de la N,N'-bis(éthyl)N,N'-bis(3'-méthyl 4'-nitrosophényl)éthylènediamine.

A une solution de 0,05 mole (18,5 g) de N,N'-bis(éthyl)N,N'-bis(3'-méthylphényl)éthylène diamine, sous forme de dichlorhydrate préparée à l'étape précédente, de 20 ml d'acide chlorhydrique concentré et de 105 g de glace, on ajoute goutte à goutte à 0°C une solution de 0,106 mole (7,3 g) de nitrite de sodium dans 17 ml d'eau. L'agitation est maintenue 30 minutes après la fin de la coulée. Le chlorhydrate du produit attendu cristallise.

Par ajout de 15 ml d'ammoniaque à 20% à une suspension du produit obtenu dans 200 ml d'eau, le produit attendu précipite. Il est recristallisé de 200 ml d'éthanol à 96° et il fond à 157°C.

L'analyse du produit obtenu donne les résultats suivantes :

Analyse pour $C_{20}H_{26}N_4O_2$

| | C% | H% | N% | O% |
|---|---|---|---|---|
| Calculé | 67,77 | 7,39 | 15,81 | 9,03 |
| Trouvé | 68,03 | 7,42 | 16,06 | 9,16 |

3ème étape :

Préparation de l'hydrate du tétrachlorhydrate de N,N'-bis(éthyl)N,N'-bis-(4'-amino 3'-méthylphényl)éthylène diamine.

A 60 ml d'alcool éthylique à 96° additionné de 8 ml d'eau, de 0,8 g de chlorure d'ammonium et de 31 g de zinc en poudre fine portés au reflux, on ajoute par portions 0,039 mole (14,1 g) du dérivé dinitrosé préparé à l'étape précédente. Le chauffage est maintenu 30 minutes après la fin de la coulée.

Par filtration du milieu réactionnel à chaud, on élimine le zinc. Après ajout d'une solution éthanolique d'acide chlorhydrique et évaporation à sec, on obtient le produit attendu.

L'analyse du produit obtenu après purification dans l'éthanol chlorhydrique, donne les résultats suivants :

Analyse pour $C_{20}H_{36}N_4OCl_4$

| | C% | H% | N% | O% | Cl% |
|---|---|---|---|---|---|
| Calculé | 48,99 | 7,40 | 11,43 | 3,26 | 28,92 |
| Trouvé | 48,88 | 7,46 | 11,37 | 3,40 | 28,75 |

## EXEMPLES DE FORMULATION

### EXEMPLE 1

| | | | |
|---|---|---|---|
| - N,N'-bis(ß-hydroxyéthyl)N,N'-bis [(4-amino)phényl]éthylènediamine, (dichlorhydrate) | | 0,15 | g |
| - Paraphénylènediamine | | 0,15 | g |
| - Polyéthylèneglycol 300 | | 10,0 | g |
| - Laurylsulfate d'ammonium | | 16,0 | g |
| - Alcools $C_{12}$-$C_{14}$ oxyéthylénés à 12 moles d'oxyde d'éthylène | | 4,0 | g |
| - Sel pentasodique de l'acide diéthylène triamine pentacétique | | 0,2 | g |
| - Métabisulfite de sodium | | 0,4 | g |
| - Résorcine | | 0,3 | g |
| - $NH_4OH$ à 20% | | 10,0 | g |
| - Eau | qsp | 100,0 | g |

Au moment de l'emploi, on mélange avec un poids égal d'eau oxygénée à 20 volumes.

Appliquée sur des cheveux sensibilisés de façon inégale sur toute leur longueur, pendant 30 minutes, on obtient, après rinçage, lavage et séchage, une couleur châtain foncé.

$$V_D - V_{DP} = -0,2$$

### EXEMPLE 2

| | | | |
|---|---|---|---|
| - Tétrachlorhydrate de N N'-bis (4'-aminophényl)1,3-diaminopropane-2-ol | | 0,15 | g |
| - Paraphénylènediamine | | 0,15 | g |
| - Polyéthylèneglycol 300 | | 10,0 | g |
| - Laurylsulfate d'ammonium | | 16,0 | g |
| - Alcools $C_{12}$-$C_{14}$ oxyéthylénés à 12 moles d'oxyde d'éthylène | | 4,0 | g |
| - Sel pentasodique de l'acide diéthylène triamine pentacétique | | 0,2 | g |
| - Métabisulfite de sodium | | 0,4 | g |
| - Résorcine | | 0,3 | g |
| - $NH_4OH$ à 20% | | 10,0 | g |
| - Eau | qsp | 100,0 | g |

Au moment de l'emploi, on ajoute un poids égal d'eau oxygénée à 20 volumes.

Cette composition, appliquée sur des cheveux sensibilisés de façon inégale sur toute leur longueur, pendant 30 minutes, leur confère, après rinçage et lavage, une couleur châtain foncé.

$$V_D - V_{DP} = +0,1$$

EXEMPLE 3

| | | | |
|---|---|---|---|
| - N,N'-bis(ß-hydroxyéthyl)N,N'-bis (4'-aminophényl)1,3-diaminopropane-2-ol (tétrachlorhydrate) | | 0,15 | g |
| - Paraphénylènediamine | | 0,15 | g |
| - Polyéthylèneglycol 300 | | 10,0 | g |
| - Laurylsulfate d'$NH_4$ | | 16,0 | g |
| - Alcools $C_{12}$-$C_{14}$ oxyéthylénés à 12 moles d'oxyde d'éthylène | | 4,0 | g |
| - Sel pentasodique de l'acide diéthylène triamine pentacétique | | 0,2 | g |
| - Métabisulfite de sodium | | 0,4 | g |
| - Résorcine | | 0,3 | g |
| - $NH_4OH$ à 20% | | 10,0 | g |
| - Eau | qsp | 100,0 | g |

Même mode d'application que dans les exemples 1 et 2.
Couleur châtain foncé.

$$V_D - V_{DP} = 0$$

EXEMPLE 4

Similaire à l'exemple 3, mais on remplace la paraphénylènediamine par la même quantité de paratoluylènediamine.
Couleur châtain clair.

$$V_D - V_{DP} = +0{,}4$$

EXEMPLE 5

| | | |
|---|---|---|
| - N,N'-bis(ß-hydroxyéthyl)N,N'-bis (4'-aminophényl)1,3-diaminopropane-2-ol (tétrachlorhydrate) | | 0,07 g |
| - Paraphénylènediamine | | 0,07 g |
| - Polyéthylèneglycol 300 | | 10,0 g |
| - Laurylsulfate de $NH_4$ | | 16,0 g |
| - Alcools $C_{12}$-$C_{14}$ oxyéthylénés à 12 moles d'oxyde d'éthylène | | 4,0 g |
| - Sel pentasodique de l'acide diéthylène triamine pentacétique | | 0,2 g |
| - Métabisulfite de sodium | | 0,4 g |
| - Résorcine | | 0,14 g |
| - 2-méthylrésorcine | | 0,01 g |
| - $NH_4OH$ à 20% | | 10,0 g |
| - Eau | qsp | 100,0 g |

Même mode d'application que dans les exemples 1 et 2.
Couleur blond foncé.

$$V_D - V_{DP} = +0,1$$

EXEMPLE 6

| | | |
|---|---|---|
| - Hydrate de tétrachlorhydrate de la N,N'-bis (éthyl) N,N'-bis (4'-amino 3'-méthylphényl) éthylènediamine | | 0,7 g |
| - Paraphénylènediamine | | 0,7 g |
| - Polyéthylèneglycol 300 | | 10,0 g |
| - Laurylsulfate d'ammonium | | 16,0 g |
| - Alcool $C_{12}$-$C_{14}$ oxyéthyléné à 12 moles d'oxyde d'éthylène | | 4,0 g |
| - Résorcine | | 0,14 g |
| - 2-méthyl résorcine | | 0,01 g |
| - Métabisulfite de sodium | | 0,04 g |
| - Sel pentasodique de l'acide diéthylène triamine pentacétique | | 0,2 g |
| - Ammoniaque à 20% | | 10,0 g |
| - Eau | qsp | 100,0 g |

Au moment de l'emploi, on ajoute un poids égal d'eau oxygénée à 20 volumes.

Cette composition appliquée sur des cheveux sensibilisés de façon inégale sur toute leur longueur, pendant 30 minutes, leur confère, après rinçage et lavage, une couleur brune.

$$V_D - V_{DP} = + 0,3$$

## Revendications

### Revendications pour les Etats contractants suivants : AT, BE, CH, DE, FR, GB, GR, IT, LI, NL, SE

1. Composition tinctoriale pour fibres kératiniques, et en particulier nour cheveux humains, comprenant, dans un milieu approprié pour la teinture de ces fibres :
(a) au moins un précurseur de colorant appartenant à la famille (A) des bases simples choisies parmi les naraphénylènediamines de formule :

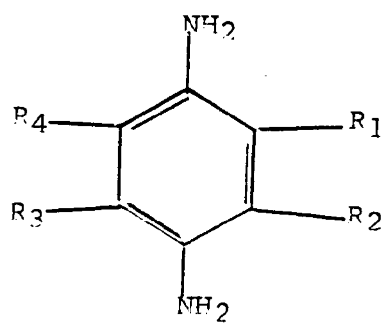

(I)

dans laquelle $R_1$, $R_2$, $R_3$, $R_4$, indépendamment l'un de l'autre, représentent un atome d'hydrogène, d'halogène, un radical alkyle ou alcoxy; ainsi que les sels de ces composés;
(b) au moins un précurseur de colorant appartenant à la famille (B) des bases doubles choisies parmi les N,N'-diphénylalcoylènediamines, répondant à la formule :

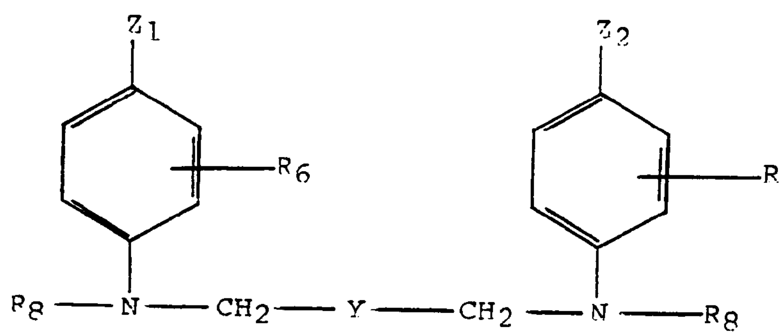

(II)

dans laquelle :
$Z_1$ et $Z_2$, identiques ou différents, représentent des groupements hydroxyle ou $NHR_9$, dans lequel $R_9$ désigne un atome d'hydrogène ou un radical alkyle;
$R_6$ et $R_7$, identiques ou différents, représentent, soit des atomes d'hydrogène, soit des atomes d'halogène, soit encore des groupements alkyle;
$R_8$ représente un atome d'hydrogène, un groupement alkyle, hydroxyalkyle ou aminoalkyle, le reste amino peut être substitué par un groupement alkyle;
Y représente un radical pris dans le groupe constitué par les radicaux suivants :

$$-(CH_2)_n- ; \quad -(CH_2)_{n'}-O-(CH_2)_{n'}- ;$$
$$-(CH_2)_{n'}-CHOH-(CH_2)_{n'}- ; \quad -(CH_2)_{n'}-\underset{\substack{|\\CH_3}}{N}-(CH_2)_{n'}- ;$$

n étant un nombre entier compris entre 0 et 8, et
n' étant un nombre entier compris entre 0 et 4,
ainsi que leurs sels d'addition avec des acides;

caractérisée par le fait que (a) les précurseurs de colorants des familles (A) et (B) sont choisis de façon à ce que l'intensité de la coloration sur cheveux décolorés teints ($V_D$) et l'intensité sur cheveux décolorés permanentés teints ($V_{DP}$), soit telle que $V_D$-$V_{DP}$=0±0,5, les valeurs d'intensité ou "value" étant déterminées selon la notation MUNSELL; (b) que le rapport molaire entre les bases simples de la famille (A)et les bases doubles de la famille (B) est compris entre 3 et 10; (c) que la composition ne contient pas d'ions iodures exceptés ceux présents naturellement dans l'eau.

2. Composition selon la revendication 1, caractérisée par le fait que dans les formules (I) et (II), les groupements alkyle et alcoxy ont de 1 à 4 atomes de carbone, le groupement hydroxyalkyle a de 2 à 4 atomes de carbone.

3. Composition selon la revendication 1 ou 2, caractérisé par le fait que les composés de formule (I) sont choisis parmi la paraphénylènediamine, la paratoluylènediamine, la 2,6-diméthylparaphénylènediamine, la 2,5-diméthylparaphénylènediamine, la 2,3-diméthylparaphénylènediamine, la 2-chloroparaphénylènediamine, la 2-méthoxyparaphénylènediamine, la 2-α-hydroxyméthylparaphénylènediamine, la 2-β-hydroxyéthylparaphénylènediamine, le 2-isopropylparaphénylènediamine, la 2,6-diméthyl 3-méthoxyparaphénylènediamine.

4. Composition selon la revendication 3, caractérisée par le fait que le composé de formule (I) est la paraphénylènediamine.

5. Composition selon l'une quelconque des revendications 1 à 4, caractérisée par le fait que les composés de formule (II) sont choisis parmi la N,N'-bis-[(4-amino)phényl]tétraméthylènediamine, la N,N'-bis-(β-diéthylaminoéthyl)N,N'-bis-[(4-amino)phényl]tétraméthylènediamine, la N-(4-hydroxy)phényl N'[(4'-amino) phényl]éthylènediamine, la N,N'-bis-(β-hydroxyéthyl) N,N'-bis-(4-aminophényl)éthylènediamine, la N,N'-bis-(4'-aminophényl)1,3-diaminopropane-2-ol, la N,N'-bis-(β-hydroxyéthyl)N,N'-bis-(4'-aminophényl),3-diaminopropane-2-ol, la N,N'-bis(éthyl)N,N'-bis(4'-amino 3'-méthylphényl)éthylènedimaine.

6. Composition selon l'une quelconque des revendications 1 à 5, caractérisée par le fait que les précurseurs de colorants des familles (A) et (B) sont présents à une concentration totale inférieure ou égale à 6% par rapport au poids total de la composition et de préférence comprise entre 0,01 et 6%.

7. Composition selon l'une quelconque des revendications 1 à 6, caractérisée par le fait que le rapport molaire entre les bases simples de la famille (A) et les bases doubles de la famille (B) est compris entre 3,5 et 7.

8. Composition selon l'une quelconque des revendications 1 à 7, caractérisée par le fait qu'elle contient également des coupleurs choisis parmi les phénols, les métadiphénols, les métaaminophénols, les métaphénylènediamines, les métaacylaminophénols, les méta-uréidophénols, les métacarbalcoxyaminophénols, l'α-naphtol, les coupleurs possédant un groupe méthylène actif, les dérivés de pyridine, les dérivés du phénol ou de l'aniline comportant un cycle méthylènedioxy.

9. Composition selon les revendications 7 ou 8, caractérisée par le fait que les coupleurs sont présents dans des proportions allant jusqu'à 10% en poids et de préférence de 0 à 6% par rapport au poids total de la composition.

10. Composition selon l'une quelconque des revendications 1 à 9, caractérisée par le fait que la composition contient également les paraphénylènediamines comportant un groupement amine tertiaire ou secondaire, du p-aminophénol ou ses dérivés substitués sur le noyau benzénique ou sur la fonction amine, des dérivés de la pyridine ou de la pyrimidine, ainsi que leurs mélanges.

11. Composition selon l'une quelconque des revendications 1 à 10, caractérisée par le fait qu'elle contient également des colorants directs appartenant aux familles des colorants benzéniques nitrés, des colorants anthraquinoniques et des colorants azoïques, présents dans des proportions pouvant aller jusqu'à 5% en poids par rapport au poids total de la composition.

12. Composition selon l'une quelconque des revendications 1 à 11, caractérisée par le fait qu'elle se présente sous forme de liquides plus ou moins épaissis, de liquide gélifiable par dilution, de crèmes plus ou moins épaissies, éventuellement conditionnées en aérosol.

13. Composition selon l'une quelconque des revendications 1 à 12, caractérisée par le fait que le milieu approprié pour la teinture est un milieu aqueux constitué par de l'eau ou un mélange eau-solvant(s) dans lequel les solvants sont présents dans des concentrations comprises entre 0,5 et 75% par rapport au poids total de la composition.

14. Composition selon les revendications 1 à 13, caractérisé par le fait qu'elle contient également des agents tensio-actifs présents dans des proportions comprises entre 0,1 et 50% en poids.

15. Composition selon l'une quelconque des revendications 1 à 14, caractérisé par le fait qu'elle contient en plus des agents épaississants présents dans des proportions comprises entre 0,1 et 5% en poids par rapport au poids total de la composition.

16. Composition selon l'une quelconque des revendications 1 à 15, caractérisée par le fait qu'elle contient également des parfums, des agents conservateurs, des agents alcalinisants ou acidifiants, des cires, des corps gras, des agents traitants du cheveu, des agents séquestrants, des agents réducteurs.

17. Composition selon l'une quelconque des revendications 1 à 16, conditionnée sous pression, caractérisée par le fait qu'elle comprend un générateur de mousse et un agent propulseur en vue de former une mousse.

18. Procédé de teinture des fibres kératiniques, en particulier des cheveux humains, caractérisé par le fait que l'on applique sur ces fibres, dans un milieu approprié pour la teinture :

    (a) au moins un précurseur de colorant appartenant à la famille des bases simples (A) définies dans la revendication 1, et

    (b) au moins un précurseur de colorant appartenant à la famille des bases doubles (B), répondant à la formule (II), définies dans la revendication 1, en présence d'un agent oxydant; le rapport molaire entre les bases simples de la famille (A) et les bases doubles de la famille (B) étant compris entre 3 et 10.

19. Procédé selon la revendication 18, caractérisé par le fait que les précurseurs de colorants de la famille (A) et les précurseurs de colorants de la famille (B) sont appliqués dans des étapes séparées sur les fibres kératiniques.

20. Procédé selon la revendication 18, caractérisé par le fait que les précurseurs de colorants des familles (A) et (B) sont appliqués sur les fibres kératiniques au moyen d'une composition telle que définie dans l'une quelconque des revendications 1 à 17.

21. Procédé selon la revendication 18, caractérisé par le fait que la composition définie dans l'une quelconque des revendications 1 à 17, est préparée tout juste avant l'emploi.

22. Dispositif à plusieurs compartiments ou kit de teinture, caractérisé par le fait qu'il comprend, dans un premier compartiment, une composition contenant dans un milieu approprié pour la teinture, au moins un précurseur de colorant de la famille (A) des bases simples définies dans la revendication 1, et dans un second compartiment, une composition comprenant dans un milieu approprié pour la teinture, au moins un précurseur de colorant de la famille (B) des bases doubles, répondant à la formule (II), définies dans la revendication 1, le rapport molaire entre le précurseur du colorant de la famille (A) et le précurseur du colorant de la famille (B) est compris entre 3 et 10.

**Revendications pour l'Etat contractant suivant : ES**

1. Procédé de préparation d'une composition tinctoriale pour fibres kératiniques et en particulier pour cheveux humains, comprenant l'association dans un milieu aqueux constitué par l'eau ou par un mélange eau-solvant(s) :

    (a) d'au moins un précurseur de colorant appartenant à la famille (A) des bases simples choisies parmi les paraphénylènediamines de formule :

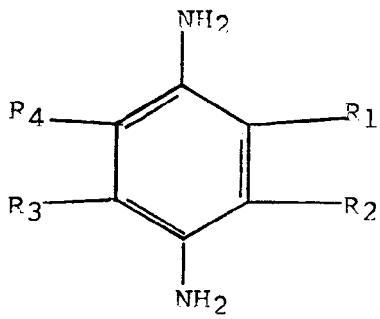

(I)

dans laquelle $R_1$, $R_2$, $R_3$, $R_4$, indépendamment l'un de l'autre, représentent un atome d'hydrogène, d'halogène, un radical alkyle ou alcoxy; ainsi que les sels de ces composés;
(b) d'au moins un précurseur de colorant appartenant à la famille (B) des bases doubles choisies parmi les N,N'-diphénylalcoylènediamines, répondant à la formule :

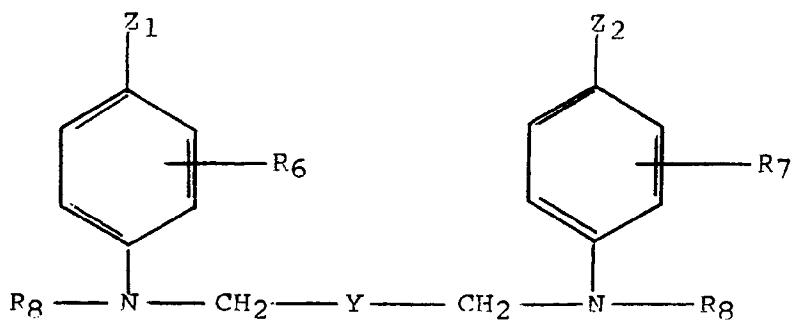

(II)

dans laquelle :
$Z_1$ et $Z_2$, identiques ou différents, représentent des groupements hydroxyle ou $NHR_9$, dans lequel $R_9$ désigne un atome d'hydrogène ou un radical alkyle;
$R_6$ et $R_7$, identiques ou différents, représentent, soit des atomes d'hydrogène, soit des atomes d'halogène, soit encore des groupements alkyle;
$R_8$ représente un atome d'hydrogène, un groupement alkyle, hydroxyalkyle ou aminoalkyle, le reste amino peut être substitué par un groupement alkyle;
Y représente un radical pris dans le groupe constitué par les radicaux suivants :

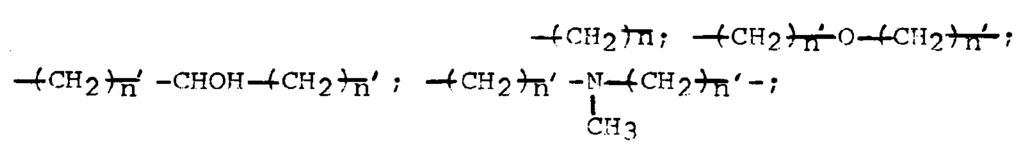

n étant un nombre entier compris entre 0 et 8, et
n' étant un nombre entier compris entre 0 et 4,
ainsi que leurs sels d'addition avec des acides;
caractérisé par le fait que :

(i) le précurseurs de colorants des familles (A) et (B) sont choisi de façon à ce que l'intensité de la coloration sur cheveux décolorés teints ($V_D$) et l'intensité sur cheveux décolorés permanentés teints ($V_{DP}$), soit telle que $V_D$-$V_{DP}$=0±0,5, les valeurs d'intensité ou "value" étant déterminées selon la notation MUNSELL;
(ii) le rapport molaire entre les bases simples de la famille (A) et les bases doubles de la famille (B) est compris entre 3 et 10;
(iii) la composition ne contient pas d'ions iodures, excepté ceux présents naturellement dans l'eau.

**2.** Procédé selon la revendication 1, caractérisé par le fait que dans les formules (I) et (II), les groupements alkyle et alcoxy ont de 1 à 4 atomes de carbone, le groupement hydroxyalkyle a de 2 à 4 atomes de carbone et le groupement aminoalkyle de 1 à 4 atomes de carbone.

3. Procédé selon la revendication 1 ou 2, caractérisé par le fait que les composés de formule (I) sont choisis parmi la paraphénylènediamine, la paratoluylènediamine, la 2,6-diméthylparaphénylènediamine, la 2,5-diméthylparaphénylènediamine, la 2,3-diméthylparaphénylènediamine, la 2-chloroparaphénylènediamine, la 2-méthoxyparaphénylènediamine, la 2-α-hydroxyméthylparaphénylènediamine, la 2-β-hydroxyéthylparaphénylènediamine, la 2-isopropylparaphénylènediamine, la 2,6-diméthyl 3-méthoxyparaphénylènediamine.

4. Procédé selon la revendication 3, caractérisé par le fait que le composé de formule (I) est la paraphénylènediamine.

5. Procédé selon les revendications 1 à 4, caractérisé par le fait que les composés de formule (II) sont choisis parmi la N,N′-bis-[(4-amino)phényl] tétraméthylènediamine, la N,N′-bis-(β-diéthylaminoéthyl) N,N′-bis-[(4-amino)phényl]tétraméthylènediamine, la N-(4-hydroxy)phényl N′[(4′-amino)phényl]éthylènediamine, la N,N′-bis-(β-hydroxyéthyl)N,N′-bis-(4-aminophényl) éthylènediamine, la N,N′-bis-(4′-aminophényl)1,3-diaminopropane-2-ol, la N,N′-bis-(β-hydroxyéthyl)N,N′-bis-(4′-aminophényl)1,3-diaminopropane-2-ol, la N,N-bis (éthyl)N,N′-bis(4′-amino 3′-méthylphényl)éthylène diamine.

6. Procédé selon les revendications 1 à 5, caractérisé par le fait que les précurseurs de colorants des familles (A) et (B) sont présents à une concentrations totale inférieure ou égale à 6% par rapport au poids total de la composition et de préférence comprise entre 0,01 et 6%

7. Procédé selon les revendications 1 à 6, caractérisé par le fait que le rapport molaire entre les bases simples de la famille (A) et les bases doubles de la famille (B) est compris entre 3,5 et 7.

8. Procédé selon les revendications 1 à 7, caractérisé par le fait qu'on introduit dans la composition également des coupleurs choisis parmi les phénols, les métadiphénols, les métaaminophénols, les métaphénylènediamines, les métaacylaminophénols, les méta-ureidophénols, les métacarbalcoxyaminophénols, l'α-naphtol, les coupleurs possédant un groupe méthylène actif, les dérivés de pyridine, les dérivés du phénol ou de l'aniline comportant un cycle méthylènedioxy.

9. Procédé selon la revendication 8, caractérisé par le fait que les coupleurs sont présents dans des proportions allant jusqu'à 10% en poids et de préférence de 0 à 6% par rapport au poids total de la composition.

10. Procédé selon les revendications 1 à 9, caractérisé par le fait qu'on introduit dans la composition également des paraphénylènediamines comportant un groupement amine tertiaire ou secondaire, du p-aminophénol ou ses dérivés substitués sur le noyau benzénique ou sur la fonction amine, et/ou des dérivés de la pyridine ou de la pyrimidine, ainsi que leurs mélanges.

11. Procédé selon les revendications 1 à 10, caractérisé par le fait qu'on introduit dans la composition également des colorants directs appartenant aux familles des colorants benzéniques nitrés, des colorants anthraquinoniques et des colorants azoïques, présents dans des proportions pouvant aller jusqu'à 5% en poids par rapport au poids total de la composition.

12. Procédé selon les revendications 1 à 11, caractérisé par le fait qu'on prépare une composition se présentant sous forme de liquides plus ou moins épaissis, le liquide gélifiable par dilution, de crèmes plus ou moins épaissies, éventuellement conditionnées en aérosol.

13. Procédé selon les revendications 1 à 12, caractérisé par le fait que le milieu aqueux constitué par un mélange eau-solvant(s), comprend des solvants dans des concentrations comprises entre 0,5 et 75% par rapport au poids total de la composition.

14. Procédé selon les revendications 1 à 13, caractérisé par le fait qu'on introduit dans la composition également des agents tensio-actifs dans des proportions comprises entre 0,1 et 50% en poids.

15. Procédé selon les revendications 1 à 14, caractérisé par le fait qu'on introduit dans la composition également des agents épaississants dans des proportions comprises entre 0,1 et 5% en poids par rapport au poids total de la composition.

16. Procédé selon les revendications 1 à 15, caractérisé par le fait qu'on introduit dans la composition également au moins un adjuvant choisi parmi des parfums, des agents conservateurs, des agents alcalinisants ou acidifiants, des cires, des corps gras, des agents traitants du cheveu, des agents séquestrants, des agents réducteurs.

**17.** Procédé selon les revendications 1 à 16, pour préparer une composition conditionnée sous pression, caractérisé par le fait qu'on y introduit également un générateur de mousse et un agent propulseur en vue de former une mousse.

**18.** Composition tinctoriale pour fibres kératiniques, et en particulier pour cheveux humains, préparée selon l'une quelconque des revendications 1 à 17.

**19.** Composition tinctoriale pour la teinture des fibres kératiniques, et en particulier pour cheveux humains, comprenant dans un milieu aqueux approprié pour la teinture de ces fibres :

(a) au moins un précurseur de colorant appartenant à la famille (A) des bases simples choisies parmi les paraphénylènediamines de formule :

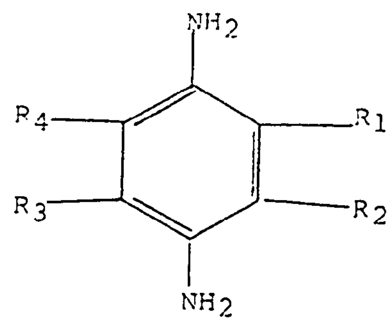

(I)

dans laquelle $R_1$, $R_2$, $R_3$, $R_4$, indépendamment l'un de l'autre, représentent un atome d'hydrogène, d'halogène, un radical alkyle ou alcoxy; ainsi que les sels de ces composés;

(b) au moins un précurseur de colorant appartenant à la famille (B) des bases doubles choisies parmi les N,N'-diphénylalcoylènediamines, répondant à la formule :

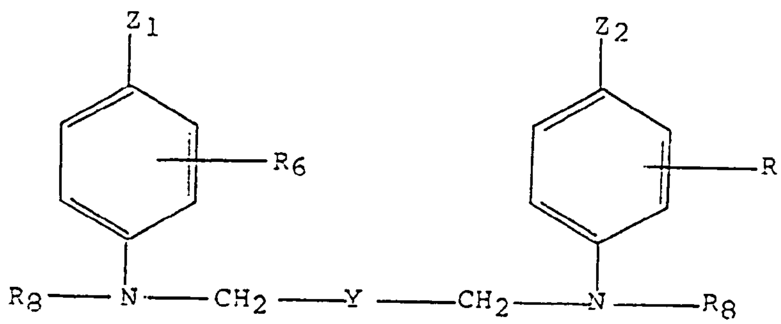

(II)

dans laquelle :

$Z_1$ et $Z_2$, identiques ou différents, représentent des groupements hydroxyle ou $NHR_9$, dans lequel $R_9$ désigne un atome d'hydrogène ou un radical alkyle;

$R_6$ et $R_7$, identique ou différents, représentent, soit des atomes d'hydrogène, soit des atomes d'halogène, soit encore des groupements alkyle;

$R_8$ représente un atome d'hydrogène, un groupement alkyle, hydroxyalkyle ou aminoalkyle, le reste amino pouvant être substitué par un groupement alkyle;

Y représente un radical pris dans le groupe constitué par les radicaux suivants :

$-(CH_2)_n-$ ; $-(CH_2)_{n'}-O-(CH_2)_{n'}-$ ;

$-(CH_2)_{n'}-CHOH-(CH_2)_{n'}-$ ; $-(CH_2)_{n'}-N(CH_3)-(CH_2)_{n'}-$ ;

dans lesquels :
n est un nombre entier compris entre 0 et 8, et
n' est un nombre entier compris entre 0 et 4,
ainsi que leurs sels d'addition avec des acides;

les précurseurs de colorants des familles (A) et (B) sont choisis de façon à ce que l'intensité de la coloration sur cheveux décolorés teints ($V_D$) et l'intensité sur cheveux décolorés permanentés teints ($V_{DP}$), soit telle que $V_D$-$V_{DP}$=0±0,5, les valeurs d'intensité ou "value" étant déterminées selon la notation MUNSELL; le rapport molaire entre les bases simples de la famille (A) et les bases doubles de la famille (B) est compris entre 3 et 10, la composition ne contient pas d'ions iodures, excepté ceux présents naturellement dans l'eau.

**20.** Composition selon la revendication 19, caractérisé par le fait que les composés de formule (I) sont choisis parmi la paraphénylènediamine, la paratoluylènediamine, la 2,6-diméthylparaphénylènediamine, la 2,5-diméthylparaphénylènediamine, la 2,3-diméthylparaphénylènediamine, la 2-chloroparaphénylènediamine, la 2-méthoxyparaphénylènediamine, la 2-α-hydroxyméthylparaphénylènediamine, la 2-β-hydroxyéthylparaphénylènediamine, la 2-isopropylparaphénylènediamine, la 2,6-diméthyl 3-méthoxyparaphénylènediamine.

**21.** Composition selon la revendication 20, caractérisée par le fait que le composé de formule (I) est la paraphénylènediamine.

**22.** Composition selon les revendications 18 à 21, caractérisée par le fait que les composés de formule (II) sont choisis parmi la N,N'-bis-[(4-amino)phényl] tétraméthylènediamine, la N,N'-bis-(β-diéthylaminoéthyl) N,N'-bis-[(4-amino)phényl]tétraméthylènediamine, la N-(4-hydroxy)phényl N'[4'-amino)phényl]éthylènediamine, la N,N'-bis-(β-hydroxyéthyl)N,N'-bis-(4-aminophényl) éthylènediamine, la N,N'-bis-(4'-aminophényl)1,3-diaminopropane-2-ol, la N,N'-bis-(β-hydroxyéthyl)N,N'-bis-(4'-aminophényl)1,3-diaminopropane-2-ol, la N,N-bis (éthyl)N,N'-bis(4'-amino 3'-méthylphényl)éthylène diamine.

**23.** Procédé de teinture des fibres kératiniques, en particulier des cheveux humains, caractérisé par le fait que l'on applique sur ces fibres, dans un milieu approprié pour la teinture :
(a) au moins un précurseur de colorant appartenant à la famille des bases simples (A) définies dans la revendication 1, et
(b) au moins un précurseur de colorant appartenant à la famille des bases doubles (B), répondant à la formule (II), définies dans la revendication 1, en présence d'un agent oxydant; le rapport molaire entre les bases simples de la famille (A) et les bases de la famille (B) étant compris entre 3 et 10.

**24.** Procédé selon la revendication 23, caractérisé par le fait que les précurseurs de colorants de la famille (A) et les précurseurs de colorants de la famille (B) sont appliqués dans des étapes séparées sur les fibres kératiniques.

**25.** Procédé selon la revendication 23, caractérisé par le fait que les précurseurs de colorants des familles (A) et (B) sont appliqués sur les fibres kératiniques au moyen d'une composition telle que définie dans l'une quelconque des revendications 18 à 22.

**26.** Procédé selon la revendication 23, caractérisé par le fait que la composition définie dans l'une quelconque des revendications 18 à 22, est préparée tout juste avant l'emploi.

**27.** Dispositif à plusieurs compartiments ou kit de teinture, caractérisé par le fait qu'il comprend, dans un premier compartiment, une composition contenant dans un milieu approprié pour la teinture, au moins un précurseur de colorant de la famille (A) de bases simples définies dans la revendication 1, et dans un second compartiment, une composition comprenant dans un milieu approprié pour la teinture, au moins un précurseur de colorant de la famille (B) des bases doubles, répondant à la formule (II), définies dans la revendication 1, le rapport molaire entre le précurseur de colorant de la famille (A) et le précurseur de colorant de la famille (B) est compris entre 3 et 10.

## Patentansprüche

### Patentansprüche für folgende Vertragsstaaten: AT, BE, CH, DE, FR, GB, GR, IT, LI, NL, SE

1. Färbezusamnensetzung für keratinische Fasern, und insbesondere für menschliche Haare, enthaltend in einem für die Färbung dieser Fasern angepaßten Milieu:

   (a) mindestens eine Farbstoff-Vorstufe, die zur Familie (A) der einfachen Basen gehört, ausgewählt unter p-Phenylendiaminen der Formel:

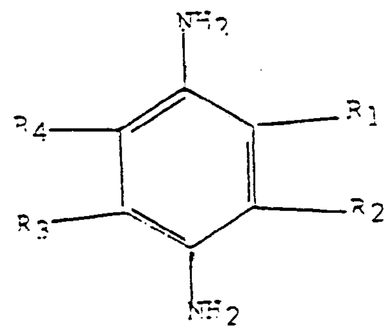

(I)

   worin $R_1$, $R_2$, $R_3$, $R_4$, unabhängig voneinander, ein Wasserstoffatom, Halogen, einen Alkyl- oder Alkoxyrest darstellen, sowie die Salze dieser Verbindungen,

   (b) mindestens eine Farbstoff-Vorstufe, die zur Familie (B) der Doppelbasen gehört, ausgewählt unter N,N'-Diphenylalkylendiaminen, gemäß der Formel:

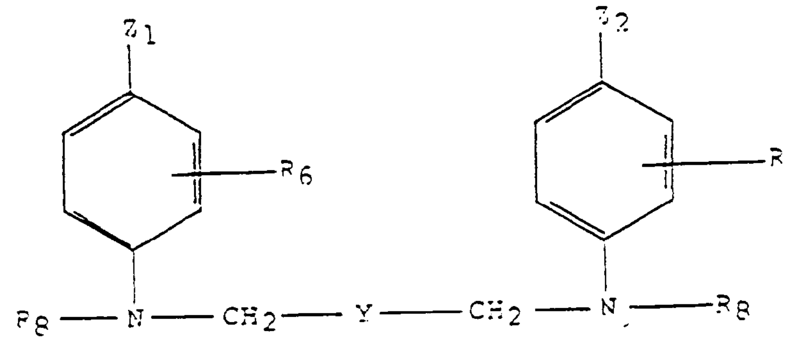

(II)

worin $Z_1$ und $Z_2$, gleich oder verschieden, Hydroxyl- oder $NHR_9$-Gruppen darstellen, worin $R_9$ ein Wasserstoffatom oder einen Alkylrest bedeutet,

$R_6$ und $R_7$, gleich oder verschieden, entweder Wasserstoffatome oder Halogenatome oder auch Alkylgruppen darstellen,

$R_8$ ein Wasserstoffatom, eine Alkyl-, Hydroxyalkyl- oder Aminoalkylgruppe darstellt, wobei der Aminorest durch eine Alkylgruppe substituiert sein kann,

Y einen Rest aus der Gruppe darstellt, bestehend aus den folgenden Resten:

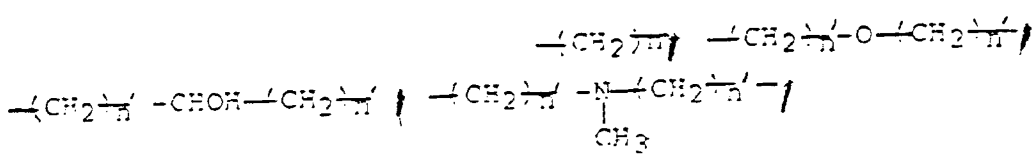

wobei n eine ganze Zahl von 0 bis 8 und

n' eine ganze Zahl von 0 bis 4 sind,

sowie deren Additionssalze mit Säuren,

dadurch **gekennzeichnet,** daß

(a) die Farbstoff-Vorstufen der Familien (A) und (B) in der Weise ausgewählt sind, daß die Intensität der Färbung auf entfärbten gefärbten Haaren ($V_D$) und die Intensität auf entfärbten gefärbten Haaren

mit Dauerwelle ($V_{DP}$) so ausfallen, daß $V_D - V_{DP} = 0 \pm 0,5$, wobei die Intensitätswerte oder der "value" gemäß der MUNSELL-Notation bestimmt sind, (b) das molare Verhältnis zwischen den einfachen Basen der Familie (A) und den Doppelbasen der Familie (B) 3 bis 10 beträgt, (c) die Zusammensetzung keine Jodionen enthält, ausser denen, die in natürlichem Wasser vorhanden sind.

**2.** Zusammensetzung gemäß Anspruch 1,
dadurch **gekennzeichnet**, daß
in den Formeln (I) und (II) die Alkyl- und Alkoxygruppen 1 bis 4 Kohlenstoffatome und die Hydroxyalkylgruppe 2 bis 4 Kohlenstoffatome aufweisen.

**3.** Zusammensetzung gemäß Anspruch 1 oder 2,
dadurch **gekennzeichnet**, daß
die Verbindungen der Formel (I) ausgewählt sind unter p-Phenylendiamin, p-Toluylendiamin, 2,6-Dimethyl-p-phenylendiamin, 2,5-Dimethyl-p-phenylendiamin, 2,3-Dimethyl-p-phenylendiamin, 2-Chlor-p-phenylendiamin, 2-Methoxy-p-phenylendiamin, 2-alpha-Hydroxymethyl-p-phenylendiamin, 2-beta-Hydroxyethyl-p-phenylendiamin, 2-Isopropyl-p-phenylendiamin, 2,6-Dimethyl-3-methoxy-p-phenylendiamin.

**4.** Zusammensetzung gemäß Anspruch 3,
dadurch **gekennzeichnet**, daß
die Verbindung der Formel (I) p-Phenylendiamin ist.

**5.** Zusammensetzung gemäß jedem Anspruch 1 bis 4,
dadurch **gekennzeichnet**, daß
die Verbindungen der Formel (II) ausgewählt sind unter
N,N′-Bis((4-amino)phenyl)tetramethylendiamin,
N,N′-Bis(beta-diethylaminoethyl)-N,N′-bis((4-amino)-phenyl)tetramethylendiamin,
N-(4-Hydroxy)phenyl-N′-((4′-amino)phenyl)ethylendiamin,
N,N′-Bis(beta-hydroxyethyl)-N,N′-bis(4-aminophenyl)-ethylendiamin, N,N′-Bis(4′-aminophenyl)-1,3-diaminopropan-2-ol,
N,N′-Bis(beta-hydroxyethyl)-N,N′-bis(4′-aminophenyl)-1,3-diaminopropan-2-ol,
N,N′-Bis(ethyl)-N,N′-bis(4′-amino-3′-methylphenyl)-ethylendiamin.

**6.** Zusammensetzung gemäß jedem Anspruch 1 bis 5,
dadurch **gekennzeichnet**, daß die Farbstoff-Vorstufen der Familien (A) und (B) in einer Gesamtkonzentration unterhalb oder gleich 6%, vorzugsweise von 0,01 bis 6%, bezogen auf Gesamtgewicht der Zusammensetzung, vorliegen.

**7.** Zusammensetzung gemäß jedem Anspruch 1 bis 6,
dadurch **gekennzeichnet**, daß
das molare Verhältnis zwischen den einfachen Basen der Familie (A) und den Doppelbasen der Familie (B) 3,5 bis 7 beträgt.

**8.** Zusammensetzung gemäß jedem der Ansprüche 1 bis 7,
dadurch **gekennzeichnet**, daß
sie auch Kuppler enthält, ausgewählt aus Phenolen, m-Diphenolen, m-Aminophenolen, m-Phenylendiaminen, m-Acylaminophenolen, m-Ureidophenolen, m-Carbalkoxyaminophenolen, alpha-Naphthol, Kupplern, die eine aktive Methylengruppe besitzen, Pyridinderivaten, Derivaten von Phenol oder Anilin, welche einen Methylendioxyring enthalten.

**9.** Zusammensetzung gemäß Ansprüchen 8,
dadurch **gekennzeichnet**, daß
die Kuppler in Mengenverhältnissen vorliegen, die bis 10 Gew.%, vorzugsweise von 0 bis 6%, bezogen auf Gesamtgewicht der Zusammensetzung, ausmachen.

**10.** Zusammensetzung gemäß jedem Anspruch 1 bis 9,
dadurch **gekennzeichnet**, daß
die Zusammensetzung auch p-Phenylendiamine mit einer tertiären oder sekundären Aminogruppe, p-Aminophenol oder seine am Benzolkern oder an der Aminofunktion substituierten Derivate, Derivate des

Pyridins oder Pyrimidins sowie deren Mischungen enthält.

**11.** Zusammensetzung gemäß jedem Anspruch 1 bis 10,
dadurch **gekennzeichnet**, daß
sie auch Direktfarbstoffe enthält, die zu den Familien der nitrierten benzolischen Farbstoffe, der Antrachinon- und Azofarbstoffe gehören, welche in Mengenverhältnissen vorliegen, die bis zu 5 Gew.%, bezogen auf Gesamtgewicht der Zusammensetzung, ausmachen können.

**12.** Zusammensetzung gemäß jedem Anspruch 1 bis 11,
dadurch **gekennzeichnet**, daß
sie in Form von mehr oder weniger verdickten Flüssigkeiten, einer durch Verdünnung gelierbaren Flüssigkeit, von mehr oder weniger verdickten Cremes, gegebenenfalls zubereitet als Aerosol, vorliegen.

**13.** Zusammensetzung gemäß jedem Anspruch 1 bis 12,
dadurch **gekennzeichnet**, daß
das für die Färbung angepaßte Milieu ein wässriges Milieu aus Wasser oder einer Mischung aus Wasser und Lösungsmittel(n) ist, worin die Lösungsmittel in Konzentrationen von 0,5 bis 75%, bezogen auf Gesamtgewicht der Zusammensetzung, vorliegen.

**14.** Zusammensetzung gemäß Anspruch 1 bis 13,
dadurch **gekennzeichnet**, daß
sie auch oberflächenaktive Mittel enthält, die in Mengenverhältnissen von 0,1 bis 50 Gew.% vorliegen.

**15.** Zusammensetzung gemäß jedem Anspruch 1 bis 14,
dadurch **gekennzeichnet**, daß
sie ausserdem Verdickungsmittel enthält, die in Mengenverhältnissen von 0,1 bis 5 Gew.%, bezogen auf Gesamtgewicht der Zusammensetzung, vorliegen.

**16.** Zusammensetzung gemäß jedem Anspruch 1 bis 15,
dadurch **gekennzeichnet**, daß
sie auch Parfüms, Konservierungsmittel, alkalisch oder sauer machende Mittel, Wachse, Fettkörper, Behandlungsmittel für das Haar, Sequestriermittel, Reduktionsmittel enthält.

**17.** Zusammensetzung gemäß jedem Anspruch 1 bis 16,
zubereitet unter Druck,
dadurch **gekennzeichnet**, daß
sie ein Schaumerzeugungsmittel und ein Treibmittel enthält, um einen Schaum zu bilden.

**18.** Verfahren zur Färbung keratinischer Fasern und insbesondere menschlicher Haare,
dadurch **gekennzeichnet**, daß
man auf die Fasern in einem für die Färbung angepaßten Milieu aufbringt:

(a) mindestens eine Farbstoff-Vorstufe, die zur Familie der in Anspruch 1 definierten einfachen Basen (A) gehört, und

(b) mindestens eine Farbstoff-Vorstufe, die zur Familie der in Anspruch 1 definierten Doppelbasen (B) gemäß der Formel (II) gehört, und zwar in Gegenwart eines Oxidationsmittels, wobei das molare Verhältnis zwischen den einfachen Basen der Familie (A) und den Doppelbasen der Familie (B) 3 bis 10 beträgt.

**19.** Verfahren gemäß Anspruch 18,
dadurch **gekennzeichnet**, daß
die Farbstoff-Vorstufen der Familie (A) und die Farbstoff-Vorstufen der Familie (B) in getrennten Stufen auf die keratinischen Fasern aufgebracht werden.

**20.** Verfahren gemäß Anspruch 18,
dadurch **gekennzeichnet**, daß
die Farbstoff-Vorstufen der Familien (A) und (B) auf die keratinischen Fasern mittels einer in jedem Anspruch 1 bis 17 definierten Zusammensetzung aufgebracht werden.

**21.** Verfahren gemäß Anspruch 18,

dadurch **gekennzeichnet**, daß
die in jedem Anspruch 1 bis 17 definierte Zusammensetzung kurz vor Gebrauch hergestellt wird.

**22.** Vorrichtung aus mehreren Bestandteilen oder Kit zur Färbung,
dadurch **gekennzeichnet**, daß
sie, in einem ersten Bestandteil, eine Zusammensetzung, enthaltend in einem zur Färbung angepaßten Milieu mindestens eine Farbstoff-Vorstufe der Familie (A) der in Anspruch 1 definierten einfachen Basen und, in einem zweiten Bestandteil, eine Zusammensetzung, enthaltend in einem zur Färbung angepaßten Milieu mindestens eine Farbstoffvorstufe der Familie (B) der in Anspruch 1 definierten Doppelbasen gemäß Formel (II) umfassen, wobei das molare Verhältnis zwischen der Farbstoff-Vorstufe der Familie (A) und der Farbstoff-Vorstufe der Familie (B) 3 bis 10 beträgt.

**Patentansprüche für folgenden Vertragsstaat : ES**

**1.** Verfahren zur Herstellung einer Färbezusammensetzung für keratinische Fasern, und insbesondere für menschliche Haare, enthaltend in einem wässrigen Milieu aus Wasser oder aus einer Mischung aus Wasser und Lösungsmittel (n) die Kombination aus:
(a) mindestens eine Farbstoff-Vorstufe, die zur Familie (A) der einfachen Basen gehört, ausgewählt unter p-Phenylendiaminen der Formel:

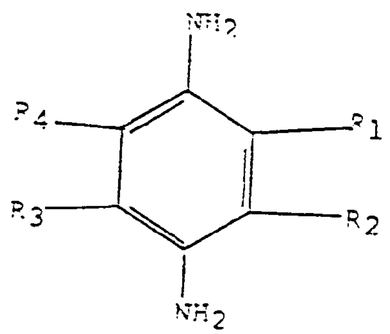

(I)

worin $R_1$, $R_2$, $R_3$, $R_4$, unabhängig voneinander, ein Wasserstoffatom, Halogen, einen Alkyl- oder Alkoxyrest darstellen, sowie die Salze dieser Verbindungen,
(b) mindestens eine Farbstoff-Vorstufe, die zur Familie (B) der Doppelbasen gehört, ausgewählt unter N,N'-Diphenylalkylendiaminen, gemäß der Formel:

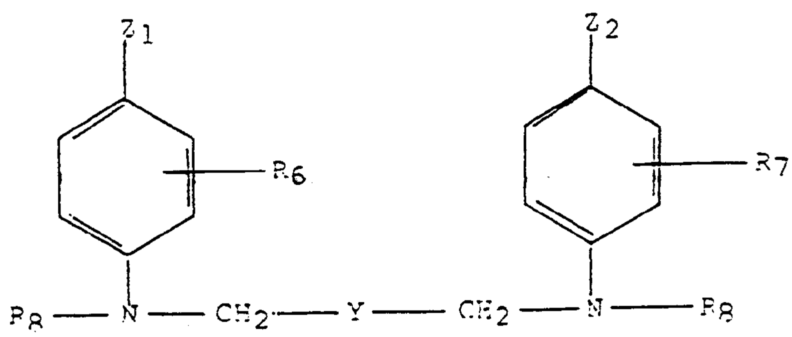

(II)

worin $Z_1$ und $Z_2$, gleich oder verschieden, Hydroxyl- oder $NHR_9$-Gruppen darstellen, worin $R_9$ ein Wasserstoffatom oder einen Alkylrest bedeutet,

$R_6$ und $R_7$, gleich oder verschieden, entweder Wasserstoffatome oder Halogenatome oder auch Alkylgruppen darstellen,

$R_8$ ein Wasserstoffatom, eine Alkyl-, Hydroxyalkyl- oder Aminoalkylgruppe darstellt, wobei der Aminorest durch eine Alkylgruppe substituiert sein kann,

Y einen Rest aus der Gruppe darstellt, bestehend aus den folgenden Resten:

$-(CH_2)_n-$ ; $-(CH_2)_{n'}-O-(CH_2)_{n'}-$ ;

$-(CH_2)_{n'}-CHOH-(CH_2)_{n'}-$ ; $-(CH_2)_{n'}-\underset{\displaystyle CH_3}{\underset{|}{N}}-(CH_2)_{n'}-$ ;

wobei n eine ganze Zahl von 0 bis 8 und n′ eine ganze Zahl von 0 bis 4 sind, sowie deren Additionssalze mit Säuren,

dadurch **gekennzeichnet**, daß

(i) die Farbstoff-Vorstufen der Familien (A) und (B) in der Weise ausgewählt sind, daß die Intensität der Färbung auf entfärbten gefärbten Haaren ($V_D$) und die Intensität auf entfärbten gefärbten Haaren mit Dauerwelle ($V_{DP}$) so ausfallen, daß $V_D - V_{DP} = 0 \pm 0{,}5$, wobei die Intensitätswerte oder der "value" gemäß der MUNSELL-Notation bestimmt sind,

(ii) das molare Verhältnis zwischen den einfachen Basen der Familie (A) und den Doppelbasen der Familie (B) 3 bis 10 beträgt,

(iii) die Zusammensetzung keine Jodionen enthält, ausser denen, die in natürlichem Wasser vorhanden sind.

2. Verfahren gemäß Anspruch 1, dadurch **gekennzeichnet**, daß in den Formeln (I) und (II) die Alkyl- und Alkoxygruppen 1 bis 4 Kohlenstoffatome und die Hydroxyalkylgruppe 2 bis 4 Kohlenstoffatome aufweisen.

3. Verfahren gemäß Anspruch 1 oder 2, dadurch **gekennzeichnet**, daß die Verbindungen der Formel (I) ausgewählt sind unter p-Phenylendiamin, p-Toluylendiamin, 2,6-Dimethyl-p-phenylendiamin, 2,5-Dimethyl-p-phenylendiamin, 2,3-Dimethyl-p-phenylendiamin, 2-Chlor-p-phenylendiamin, 2-Methoxy-p-phenylendiamin, 2-alpha-Hydroxymethyl-p-phenylendiamin, 2-beta-Hydroxyethyl-p-phenylendiamin, 2-isopropyl-p-phenylendiamin, 2,6-Dimethyl-3-methoxy-p-phenylendiamin.

4. Verfahren gemäß Anspruch 3, dadurch **gekennzeichnet**, daß die Verbindung der Formel (I) p-Phenylendiamin ist.

5. Verfahren gemäß jedem Anspruch 1 bis 4, dadurch **gekennzeichnet**, daß die Verbindungen der Formel (II) ausgewählt sind unter
N,N′-Bis((4-amino)phenyl)tetramethylendiamin,
N,N′-Bis(beta-diethylaminoethyl)-N,N′-bis((4-amino)-phenyl)tetramethylendiamin,
N-(4-Hydroxy)phenyl-N′-((4′-amino)phenyl)ethylendiamin,
N,N′-Bis(beta-hydroxyethyl)-N,N′-bis(4-aminophenyl)-ethylendiamin, N,N′-Bis(4′-aminophenyl)-1,3-diaminopropan-2-ol,
N,N′-Bis(beta-hydroxyethyl)-N,N′-bis(4′-aminophenyl)-1,3-diaminopropan-2-ol,
N,N′-Bis(ethyl)-N,N′-bis(4′-amino-3′-methylphenyl)-ethylendiamin.

6. Verfahren gemäß jedem Anspruch 1 bis 5, dadurch **gekennzeichnet**, daß die Farbstoff-Vorstufen der Familien (A) und (B) in einer Gesamtkonzentration unterhalb oder gleich 6%, vorzugsweise von 0,01 bis 6%, bezogen auf Gesamtgewicht der Zusammensetzung, vorliegen.

7. Verfahren gemäß jedem Anspruch 1 bis 6, dadurch **gekennzeichnet**, daß das molare Verhältnis zwischen den einfachen Basen der Familie (A) und den Doppelbasen der Familie (B) 3,5 bis 7 beträgt.

8. Verfahren gemäß jedem der Ansprüche 1 bis 7, dadurch **gekennzeichnet**, daß

sie auch Kuppler enthält, ausgewählt aus Phenolen, m-Diphenolen, m-Aminophenolen, m-Phenylendiaminen, m-Acylaminophenolen, m-Ureidophenolen, m-Carbalkoxyaminophenolen, alpha-Naphthol, Kupplern, die eine aktive Methylengruppe besitzen, Pyridinderivaten, Derivaten von Phenol oder Anilin, welche einen Methylendioxyring enthalten.

**9.** Verfahren gemäß Ansprüchen 7 oder 8,
dadurch **gekennzeichnet**, daß
die Kuppler in Mengenverhältnissen vorliegen, die bis 10 Gew.%, vorzugsweise von 0 bis 6%, bezogen auf Gesamtgewicht der Zusammensetzung, ausmachen.

**10.** Verfahren gemäß jedem Anspruch 1 bis 9,
dadurch **gekennzeichnet**, daß
die Zusammensetzung auch p-Phenylendiamine mit einer tertiären oder sekundären Aminogruppe, p-Aminophenol oder seine am Benzolkern oder an der Aminofunktion substituierten Derivate, Derivate des Pyridins oder Pyrimidins sowie deren Mischungen enthält.

**11.** Verfahren gemäß jedem Anspruch 1 bis 10,
dadurch **gekennzeichnet**, daß
sie auch Direktfarbstoffe enthält, die zu den Familien der nitrierten benzolischen Farbstoffe, der Antrachinon- und Azofarbstoffe gehören, welche in Mengenverhältnissen vorliegen, die bis zu 5 Gew.%, bezogen auf Gesamtgewicht der Zusammensetzung, ausmachen können.

**12.** Verfahren gemäß jedem Anspruch 1 bis 11,
dadurch **gekennzeichnet**, daß
sie in Form von mehr oder weniger verdickten Flüssigkeiten, einer durch Verdünnung gelierbaren Flüssigkeit, von mehr oder weniger verdickten Cremes, gegebenenfalls zubereitet als Aerosol, vorliegen.

**13.** Verfahren gemäß jedem Anspruch 1 bis 12,
dadurch **gekennzeichnet**, daß
das für die Färbung angepaßte Milieu ein wässriges Milieu aus Wasser oder einer Mischung aus Wasser und Lösungsmittel(n) ist, worin die Lösungsmittel in Konzentrationen von 0,5 bis 75%, bezogen auf Gesamtgewicht der Zusammensetzung, vorliegen.

**14.** Verfahren gemäß Anspruch 1 bis 13,
dadurch **gekennzeichnet**, daß
sie auch oberflächenaktive Mittel enthält, die in Mengenverhältnissen von 0,1 bis 50 Gew.% vorliegen.

**15.** Verfahren gemäß jedem Anspruch 1 bis 14,
dadurch **gekennzeichnet**, daß
sie ausserdem Verdickungsmittel enthält, die in Mengenverhältnissen von 0,1 bis 5 Gew.%, bezogen auf Gesamtgewicht der Zusammensetzung, vorliegen.

**16.** Verfahren gemäß jedem Anspruch 1 bis 15,
dadurch **gekennzeichnet**, daß
sie auch Parfüms, Konservierungsmittel, alkalisch oder sauer machende Mittel, Wachse, Fettkörper, Behandlungsmittel für das Haar, Sequestriermittel, Reduktionsmittel enthält.

**17.** Verfahren gemäß jedem Anspruch 1 bis 16,
zubereitet unter Druck,
dadurch **gekennzeichnet**, daß
sie ein Schaumerzeugungsmittel und ein Treibmittel enthält, um einen Schaum zu bilden.

**18.** Färbezusammensetzung für keratinische Fasern, und insbesondere für menschliche Haare, erhätlich gemäß jedem der Ansprüche 1 bis 17.

**19.** Färbezusammensetzung zur Färbung keratinischer Fasern, und insbesondere menschlicher Haare, enthaltend in einem für die Färbung dieser Fasern angepaßten wässrigen Milieu:
(a) mindestens eine Farbstoff-Vorstufe, die zur Familie (A) der einfachen Basen gehört, ausgewählt unter p-Phenylendiaminen der Formel:

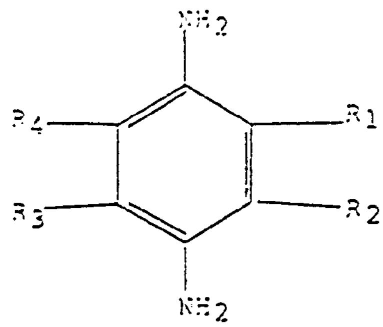

(I)

worin $R_1$, $R_2$, $R_3$, $R_4$, unabhängig voneinander, ein Wasserstoffatom, Halogen, einen Alkyl- oder Alkoxyrest darstellen, sowie die Salze dieser Verbindungen,

(b) mindestens eine Farbstoff-Vorstufe, die zur Familie (B) der Doppelbasen gehört, ausgewählt unter N,N′-Diphenylalkylendiaminen, gemäß der Formel:

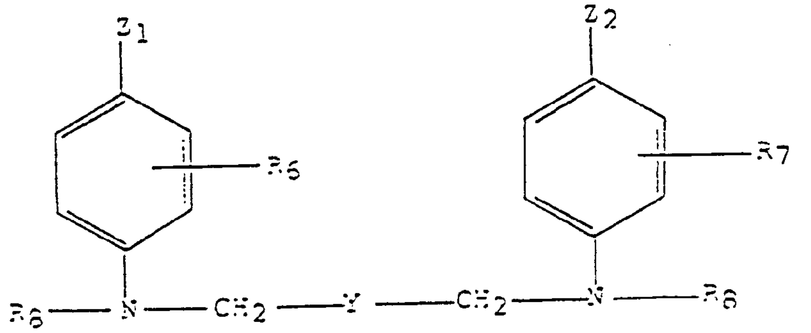

(II)

worin $Z_1$ und $Z_2$, gleich oder verschieden, Hydroxyl- oder $NHR_9$-Gruppen darstellen, worin $R_9$ ein Wasserstoffatom oder einen Alkylrest bedeutet,

$R_6$ und $R_7$, gleich oder verschieden, entweder Wasserstoffatome oder Halogenatome oder auch Alkylgruppen darstellen,

$R_8$ ein Wasserstoffatom, eine Alkyl-, Hydroxyalkyl- oder Aminoalkylgruppe darstellt, wobei der Aminorest durch eine Alkylgruppe substituiert sein kann,

Y einen Rest aus der Gruppe darstellt, bestehend aus den folgenden Resten:

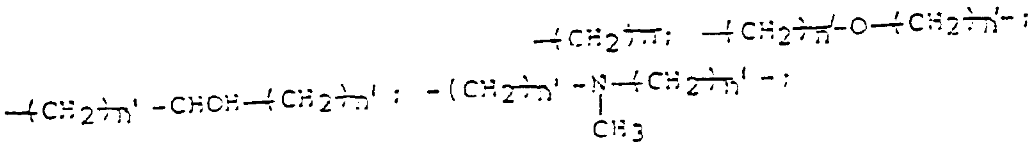

wobei n eine ganze Zahl von 0 bis 8 und

n′ eine ganze Zahl von 0 bis 4 sind,

sowie deren Additionssalze mit Säuren,

und wobei (a) die Farbstoff-Vorstufen der Familien (A) und (B) in der Weise ausgewählt sind, daß die Intensität der Färbung auf entfärbten gefärbten Haaren ($V_D$) und die Intensität auf entfärbten gefärbten Haaren mit Dauerwelle ($V_{DP}$) so ausfallen, daß $V_D - V_{DP} = 0 \pm 0{,}5$, wobei die Intensitätswerte oder der "value" gemäß der MUNSELL-Notation bestimmt sind, (b) das molare Verhältnis zwischen den einfachen Basen der Familie (A) und den Doppelbasen der Familie (B) 3 bis 10 beträgt, (c) die Zusammensetzung keine Jodionen enthält, ausser denen, die in natürlichem Wasser vorhanden sind.

**20.** Zusammensetzung gemäß Anspruch 19,
dadurch **gekennzeichnet**, daß
die Verbindungen der Formel (I) ausgewählt sind unter p-Phenylendiamin, p-Toluylendiamin, 2,6-Dimethyl-p-phenylendiamin, 2,5-Dimethyl-p-phenylendiamin, 2,3-Dimethyl-p-phenylendiamin, 2-Chlor-p-phenylendiamin, 2-Methoxy-p-phenylendiamin, 2-alpha-Hydroxymethyl-p-phenylendiamin, 2-beta-Hydroxyethyl-p-phenylendiamin, 2-Isopropyl-p-phenylendiamin, 2,6-Dimethyl-3-methoxy-p-phenylendiamin.

**21.** Zusammensetzung gemäß Anspruch 20,
dadurch **gekennzeichnet**, daß
die Verbindung der Formel (I) p-Phenylendiamin ist.

**22.** Zusammensetzung gemäß Ansprüche 18 bis 21,
dadurch **gekennzeichnet**, daß
die Verbindungen der Formel (II) ausgewählt sind unter
N,N′-Bis((4-amino)phenyl)tetramethylendiamin,
N,N′-Bis(beta-diethylaminoethyl)-N,N′-bis((4-amino)-phenyl)tetramethylendiamin,
N-(4-Hydroxy)phenyl-N′-((4′-amino)phenyl)ethylendiamin,
N,N′-Bis(beta-hydroxyethyl)-N,N′-bis(4-aminophenyl)-ethylendiamin, N,N′-Bis(4′-aminophenyl)-1,3-diaminopropan-2-ol,
N,N′-Bis(beta-hydroxyethyl)-N,N′-bis(4′-aminophenyl)-1,3-diaminopropan-2-ol,
N,N′-Bis(ethyl)-N,N′-bis(4′amino-3′-methylphenyl)-ethylendiamin.

**23.** Verfahren zur Färbung keratinischer Fasern und insbesondere menschlicher Haare,
dadurch **gekennzeichnet**, daß
man auf die Fasern in einem für die Färbung angepaßten Milieu aufbringt:
(a) mindestens eine Farbstoff-Vorstufe, die zur Familie der in Anspruch 1 definierten einfachen Basen (A) gehört, und
(b) mindestens eine Farbstoff-Vorstufe, die zur Familie der in Anspruch 1 definierten Doppelbasen (B) gemäß der Formel (II) gehört, und zwar in Gegenwart eines Oxidationsmittels, wobei das molare Verhältnis zwischen den einfachen Basen der Familie (A) und den Doppelbasen der Familie (B) 3 bis 10 beträgt.

**24.** Verfahren gemäß Anspruch 23,
dadurch **gekennzeichnet**, daß
die Farbstoff-Vorstufen der Familie (A) und die Farbstoff-Vorstufen der Familie (B) in getrennten Stufen auf die keratinischen Fasern aufgebracht werden.

**25.** Verfahren gemäß Anspruch 23,
dadurch **gekennzeichnet**, daß
die Farbstoff-Vorstufen der Familien (A) und (B) auf die keratinischen Fasern mittels einer in jedem Anspruch 18 bis 22 definierten Zusammensetzung aufgebracht werden.

**26.** Verfahren gemäß Anspruch 23,
dadurch **gekennzeichnet**, daß
die in jedem Anspruch 18 bis 22 definierte Zusammensetzung kurz vor Gebrauch hergestellt wird.

**27.** Vorrichtung aus mehreren Bestandteilen oder Kit zur Färbung,
dadurch **gekennzeichnet**, daß
sie, in einem ersten Bestandteil, eine Zusammensetzung, enthaltend in einem zur Färbung angepaßten Milieu mindestens eine Farbstoff-Vorstufe der Familie (A) der in Anspruch 1 definierten einfachen Basen und, in einem zweiten Bestandteil, eine Zusammensetzung, enthaltend in einem zur Färbung angepaßten Milieu mindestens eine Farbstoffvorstufe der Familie (B) der in Anspruch 1 definierten Doppelbasen gemäß Formel (II) umfassen, wobei das molare Verhältnis zwischen der Farbstoff-Vorstufe der Familie (A) und der Farbstoff-Vorstufe der Familie (B) 3 bis 10 beträgt.

## Claims

### Claims for the following Contracting States : AT, BE, CH, DE, FR, GB, GR, IT, LI, NL, SE

**1.** Dyeing composition for keratinous fibres, and especially for human hair, comprising, in a medium suitable for the dyeing of these fibres:
a) at least one dye precursor belonging to the family (A) of single-unit bases chosen from the paraphe-

nylenediamines of formula:

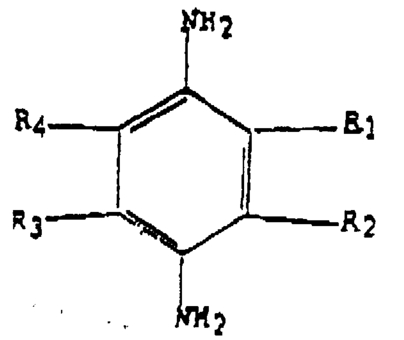

(I)

in which $R_1$, $R_2$, $R_3$ and $R_4$, independently of one another, represent a hydrogen or halogen atom or an alkyl or alkoxy radical; as well as the salts of these compounds;

b) at least one dye precursor belonging to the family (B) of double-unit bases chosen from the N,N'-diphenylalkylenediamines corresponding to the formula:

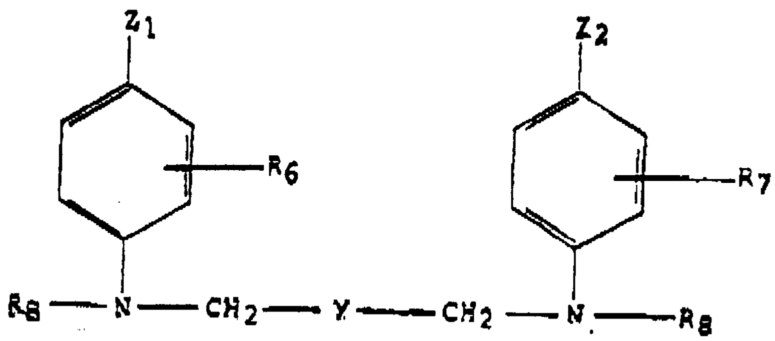

(II)

in which:

$Z_1$ and $Z_2$, which may be identical or different, represent hydroxyl groups or groups $NHR_9$ in which $R_9$ denotes a hydrogen atom or an alkyl radical;

$R_6$ and $R_7$, which may be identical or different, represent either hydrogen atoms or halogen atoms or alternatively alkyl groups;

$R_8$ represents a hydrogen atom or an alkyl, hydroxyalkyl or aminoalkyl group; the amino residue may be substituted with an alkyl group;

Y represents a radical selected from the group consisting of the following radicals:

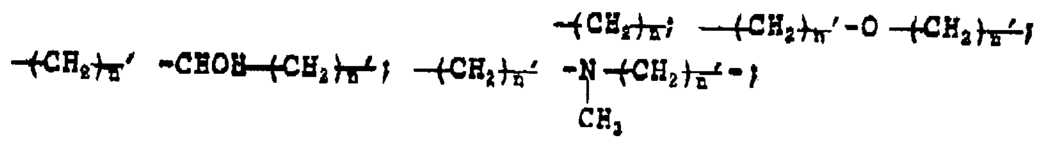

n being an integer between 0 and 8, and

n' being an integer between 0 and 4;

as well as their addition salts with acids;

characterised in that (a) the dye precursors of the families (A) and (B) are chosen so that the intensity of the coloration on dyed bleached hair ($V_D$) and the intensity on dyed, permanent-waved bleached hair ($V_{DP}$) is such that $V_D$-$V_{DP}$=0±0.5, the intensity values, or "value", being determined according to the MUNSELL notation; (b) in that the mole ratio of the single-unit bases of the family (A) to the double-unit bases of the family (B) is between 3 and 10; (c) in that the composition does not contain iodide ions except for those naturally present in the water.

2. Composition according to Claim 1, characterised in that, in the formulae (I) and (II), the alkyl and alkoxy groups have from 1 to 4 carbon atoms and the hydroxyalkyl group has from 2 to 4 carbon atoms.

3. Composition according to Claim 1 or 2, characterised in that the compounds of formula (I) are chosen from para-phenylenediamine, para-tolylenediamine, 2,6-dimethyl-para-penylenediamine, 2,5-dimethyl-para-phenylenediamine, 2,3-dimethyl-para-phenylenediamine, 2-chloro-para-phenylenediamine, 2-methoxy-para-phenylenediamine, 2-(α-hydroxymethyl)-para-phenylenediamine, 2-(β-hydroxyethyl)-para-phenylenediamine, 2-isopropyl-para-phenylenediamine and 2,6-dimethyl-3-methoxy-para-phenylenediamine.

4. Composition according to Claim 3, characterised in that the compound of formula (I) is para-phenylenediamine.

5. Composition according to any one of Claims 1 to 4, characterised in that the compounds of formula (II) are chosen from N,N′-bis(4-aminophenyl)tetramethylenediamine, N,N′-bis(β-diethylaminoethyl)-N,N′-bis(4-aminophenyl)tetramethylenediamine, N-(4-hydroxyphenyl)-N′-(4-aminophenyl)ethylenediamine, N,N′-bis(β-hydroxyethyl)-N,N′-bis(4-aminophenyl)-ethylenediamine, N,N′-bis(4-aminophenyl)-1,3-diamino-2-propanol, N,N′-bis(β-hydroxyethyl)-N,N′-bis(4-aminophenyl)-1,3-diamino-2-propanol and N,N′-bis(ethyl)-N,N′-bis(4-amino-3-methylphenyl)ethylenediamine.

6. Composition according to any one of Claims 1 to 5, characterised in that the dye precursors of the families (A) and (B) are present at a total concentration not exceeding 6% relative to the total weight of the composition, and preferably between 0.01 and 6%.

7. Composition according to any one of Claims 1 to 6, characterised in that the mole ratio of the single-unit bases of the family (A) to the double-unit bases of the family (B) is between 3.5 and 7.

8. Composition according to any one of Claims 1 to 7, characterised in that it also contains couplers chosen from phenols, meta-diphenols, meta-aminophenols, meta-phenylenediamines, meta-acylaminophenols, meta-ureidophenols, meta-carbalkoxyaminophenols, α-naphtol, couplers possessing an active methylene group, pyridine derivatives, and derivatives of phenol or of aniline containing a methylenedioxy ring.

9. Composition according to Claim 8, characterised in that the couplers are present in proportions ranging up to 10% by weight, and preferably from 0 to 6%, relative to the total weight of the composition.

10. Composition according to any one of Claims 1 to 9, characterised in that the composition also contains para-phenylenediamines containing a tertiary or secondary amine group, p-aminophenol or its derivatives substituted on the benzene ring or on the amine function, derivatives of pyridine or of pyrimidine as well as mixtures thereof.

11. Composition according to any one of Claims 1 to 10, characterised in that it also contains direct dyes belonging to the families of benzenoid nitro dyes, anthraquinone, dyes and azo dyes, present in proportions which can range up to 5% by weight relative to the total weight of the composition.

12. Composition according to any one of Claims 1 to 11, characterised in that it takes the form of more or less thickened liquids, of liquid which can be gelled on dilution, or of more or less thickened creams, where appropriate packaged as an aerosol.

13. Composition according to any one of Claims 1 to 12, characterised in that the medium suitable for dyeing is an aqueous medium consisting of water or a water/solvent(s) mixture in which the solvents are present in concentrations of between 0.5 and 75% relative to the total weight of the composition.

14. Composition according to Claims 1 to 13, characterised in that it also contains surfactants present in proportions of between 0.1 and 50% by weight.

15. Composition according to any one of Claims 1 to 14, characterised in that it contains, in addition, thickening agents present in proportions of between 0.1 and 5% by weight relative to the total weight of the composition.

16. Composition according to any one of Claims 1 to 15, characterised in that it also contains perfumes, preservatives, alkalinising or acidifying agents, waxes, fats, hair treatment agents, sequestering agents, reducing agents.

17. Composition according to any one of Claims 1 to 16, packaged under pressure, characterised in that it comprises a foam generator and a propellent agent for the purpose of forming a foam.

18. Process for dyeing keratinous fibres, especially human hair, characterised in that the following are applied on these fibres in a medium suitable for dyeing:

a) at least one dye precursor belonging to the family of single-unit bases (A) which are defined in Claim 1, and

b) at least one dye precursor belonging to the family of double-unit bases (B) corresponding to the formula (II) which are defined in Claim 1, in the presence of an oxidising agent; the more ratio of the single-unit bases of the family (A) to the double-unit bases of the family (B) being between 3 and 10.

19. Process according to Claim 18, characterised in that the dye precursors of the family (A) and the dye precursors of the family (B) are applied in separate steps on the keratinous fibres.

20. Process according to Claim 18, characterised in that the dye precursors of the families (A) and (B) are applied on the keratinous fibres by means of a composition as defined in any one of Claims 1 to 17.

21. Process according to Claim 18, characterised in that the composition defined in any one of Claims 1 to 17, is prepared immediately before use.

22. Multi-compartment device or kit for dyeing, characterised in that it comprises, in a first compartment, a composition containing, in a medium suitable for dyeing, at least one dye precursor of the family (A) of single-unit bases which are defined in Claim 1, and, in a second compartment, a composition comprising in a medium suitable for dyeing, at least one dye precursor of the family (B) of double-unit bases corresponding to the formula (II) which are defined in Claim 1; the mole ratio of the dye precursor of the family (A) to the dye precursor of the family (B) is between 3 and 10.

**Claims for the following Contracting State : ES**

1. Process for preparing a dyeing composition for keratinous fibres, and especially for human hair, comprising the combination, in an aqueous medium consisting of water or a water/solvent(s) mixture, of:

a) at least one dye precursor belonging to the family (A) of single-unit bases chosen from the para-phenylenediamines of formula:

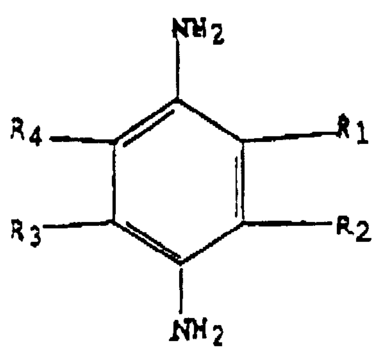

(I)

in which $R_1$, $R_2$, $R_3$ and $R_4$, independently of one another, represent a hydrogen or halogen atom of an alkyl or alkoxy radical; as well as the salts of these compounds;

b) at least one dye precursor belonging to the family (B) of double-unit bases chosen from the N,N′-diphenylalkylenediamines corresponding to the formula:

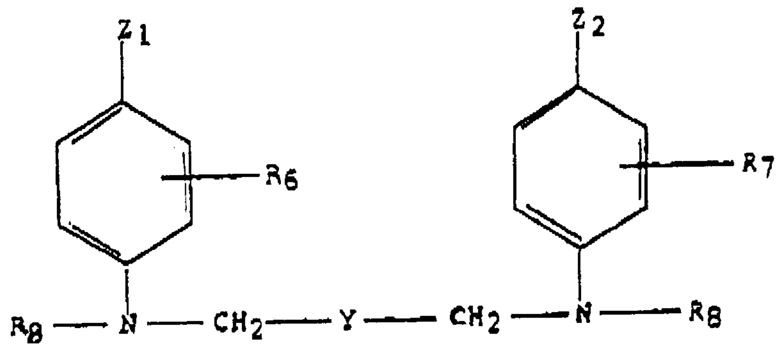

(II)

in which:

$Z_1$ and $Z_2$, which may be identical or different, represent hydroxyl groups or groups $NHR_9$ in which $R_9$ denotes a hydrogen atom or an alkyl radical;

$R_6$ and $R_7$, which may be identical of different, represent either hydrogen atoms or halogen atoms or alternatively alkyl groups;

$R_8$ represents a hydrogen atom or an alkyl, hydroxyalkyl or aminoalkyl group; the amino residue may be substituted with an alkyl group;

Y represent a radical selected from the group consisting of the following radicals:

$$-(CH_2)_n-;\quad -(CH_2)_{n'}-O-(CH_2)_{n'}-;$$
$$-(CH_2)_{n'}-CHOH-(CH_2)_{n'}-;\quad -(CH_2)_{n'}-\underset{CH_3}{\underset{|}{N}}-(CH_2)_{n'}-;$$

n being an integer between 0 and 8, and
n′ being an integer between 0 and 4;
as well as their addition salts with acids;
characterised in that:

(i) the dye precursors of the families (A) and (B) are chosen so that the intensity of the coloration on dyed bleached hair ($V_D$) and the intensity on dyed, permanent-waved bleached hair ($V_{DP}$) is such that $V_D$-$V_{DP}$=0±0.5, the intensity values, or "value", being determined according to the MUNSELL notation;
(ii) the mole ratio of the single-unit bases of the family (A) to the double-unit bases of the family (B) is between 3 and 10;
(iii) the composition does not contain iodide ions except for those naturally present in the water.

**2.** Process according to Claim 1, characterised in that, in the formulae (I) and (II), the alkyl and alkoxy groups have from 1 to 4 carbon atoms, the hydroxyalkyl group has from 2 to 4 carbon atoms and the aminoalkyl group from 1 to 4 carbon atoms.

**3.** Process according to Claim 1 or 2, characterised in that the compounds of formula (I) are chosen from para-phenylenediamine, para-tolylenediamine, 2,6-dimethyl-para-penylenediamine, 2,5-dimethyl-para-phenylenediamine, 2,3-dimethyl-para-phenylenediamine, 2-chloro-para-phenylenediamine, 2-methoxy-para-phenylenediamine, 2-(α-hydroxymethyl)-para-phenylenediamine, 2-(β-hydroxyethyl)-para-phenylenediamine, 2-isopropyl-para-phenylenediamine and 2,6-dimethyl-3-methoxy-para-phenylenediamine.

**4.** Process according to Claim 3, characterised in that the compound of formula (I) is para-phenylenediamine.

**5.** Process according to Claims 1 to 4, characterised in that the compounds of formula (II) are chosen from N,N′-bis(4-aminophenyl)tetramethylenediamine, N,N′-bis(β-diethylaminoethyl)-N,N′-bis(4-aminophenyl)tetramethylenediamine, N-(4-hydroxyphenyl)-N′-(4-aminophenyl)ethylenediamine, N,N′-bis(β-hydroxyethyl)-N,N′-bis(4-aminophenyl)-ethylenediamine, N,N′-bis(4-aminophenyl)-1,3-diamino-2-propanol, N,N′-bis(β-hydroxyethyl)-N,N′-bis(4-aminophenyl)-1,3-diamino-2-propanol and N,N′-bis(ethyl)-N,N′-bis(4-amino-3-methylphenyl)ethylenediamine.

**6.** Process according to Claims 1 to 5, characterised in that the dye precursors of the families (A) and (B)

are present at a total concentration not exceeding 6% relative to the total weight of the composition, and preferably between 0.01 and 6%.

7. Process according to Claims 1 to 6, characterised in that the mole ratio of the single-unit bases of the family (A) to the double-unit bases of the family (B) is between 3.5 and 7.

8. Process according to Claims 1 to 7, characterised in that couplers chosen from phenols, meta-diphenols, meta-aminophenols, meta-phenylenediamines, meta-acylaminophenols, meta-ureidophenols, meta-carbalkoxyaminophenols, $\alpha$-naphtol, couplers possessing an active methylene group, pyridine derivatives, and derivatives of phenol or of aniline containing a methylenedioxy ring are also introduced into the composition.

9. Process according to Claim 8, characterised in that the couplers are present in proportions ranging up to 10% by weight, and preferably from 0 to 6%, relative to the total weight of the composition.

10. Process according to Claims 1 to 9, characterised in that para-phenylenediamines containing a tertiary or secondary amine group, p-aminophenol or its derivatives substituted on the benzene ring or on the amine function and/or derivatives of pyridine or of pyrimidine, as well as mixtures thereof, are also introduced into the composition.

11. Process according to Claims 1 to 10, characterised in that direct dyes belonging to the families of benzenoid nitro dyes, anthraquinone dyes and azo dyes, present in proportions which can range up to 5% by weight relative to the total weight of the composition, are also introduced into the composition.

12. Process according to Claims 1 to 11, characterised in that a composition which takes the form of more or less thickened liquids, of liquids which can be gelled on dilution, or of more or less thickened creams, where appropriate packaged as an aerosol, is prepared.

13. Process according to Claims 1 to 12, characterised in that the aqueous medium consisting of a water/solvent(s) mixture comprises solvents in concentrations of between 0.5 and 75% relative to the total weight of the composition.

14. Process according to Claims 1 to 13, characterised in that surfactants are also introduced into the composition, in proportions of between 0.1 and 50% by weight.

15. Process according to Claims 1 to 14, characterised in that thickening agents are also introduced into the composition, in proportions of between 0.1 and 5% by weight relative to the total weight of the composition.

16. Process according to Claims 1 to 15, characterised in that at least one adjuvant chosen from perfumes, preservatives alkalinising or acidifying agents, waxes, fats, hair treatment agents, sequestering agents and reducing agents is also introduced into the composition.

17. Process according to Claims 1 to 6, for preparing a composition packaged under pressure, characterised in that a foam generator and a propellent agent are also introduced into it for the purpose of forming a foam.

18. Dyeing composition for keratinous fibres, and especially for human hair, prepared according to any one of Claims 1 to 17.

19. Dyeing composition for the dyeing of keratinous fibres, and especially for human hair, comprising, in an aqueous medium suitable for the dyeing of these fibres:
a) at least one dye precursor belonging to the family (A) of single-unit bases chosen from the para-phenylenediamines of formula:

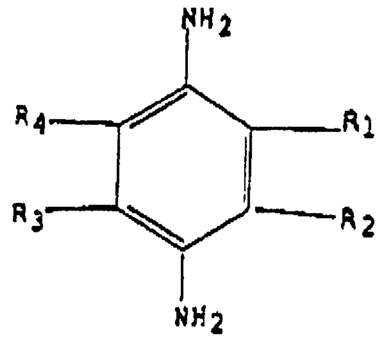

(I)

in which $R_1$, $R_2$, $R_3$ and $R_4$, independently of one another, represent a hydrogen or halogen atom or an alkyl or alkoxy radical; as well as the salts of these compounds;

b) at least one dye precursor belonging to the family (B) of double-unit bases chosen from the N,N'-diphenylalkylenediamines corresponding to the formula:

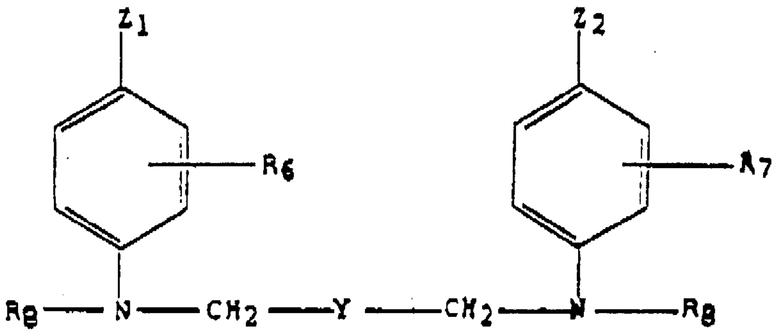

(II)

in which:

$Z_1$ and $Z_2$, which may be identical or different, represent hydroxyl groups or groups $NHR_9$ in which $R_9$ denotes a hydrogen atom or an alkyl radical;

$R_6$ and $R_7$, which may be identical or different, represent either hydrogen atoms or halogen atoms or alternatively alkyl groups;

$R_8$ represents a hydrogen atom or an alkyl, hydroxyalkyl or aminoalkyl group, it being possible for the amino residue to be substituted with an alkyl group;

Y represents a radical selected from the group consisting of the following radicals:

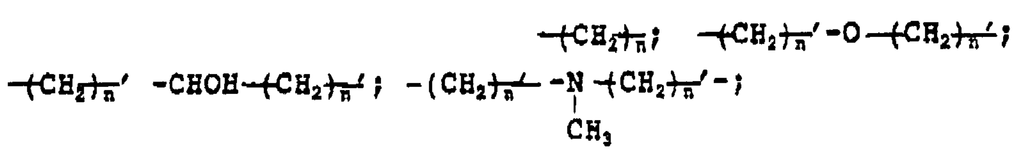

in which:

n is an integer between 0 and 8, and

n' is an integer between 0 and 4;

as well as their addition salts with acids;

the dye precursors of the families (A) and (B) are chosen so that the intensity of the coloration on dyed bleached hair ($V_D$) and the intensity on dyed, permanent-waved bleached hair ($V_{DP}$) is such that $V_D$-$V_{DP}$=0±0.5, the intensity values, or "values", being determined according to the MUNSELL notation; the mole ratio of the single-unit bases of the family (A) to the double-unit bases of the family (B) is between 3 and 10; the composition does not contain iodide ions except for those naturally present in the water.

**20.** Composition according to Claim 19, characterised in that the compounds of formula (I) are chosen from para-phenylenediamine, para-tolylenediamine, 2,6-dimethyl-para-phenylenediamine, 2,5-dimethyl-para-phenylenediamine, 2,3-dimethyl-para-phenylenediamine, 2-chloro-para-phenylenediamine, 2-methoxy-para-phenylenediamine, 2-(α-hydroxymethyl)-para-phenylenediamine, 2-(β-hydroxyethyl)-para-phenylenediamine, 2-isopropyl-para-phenylenediamine and 2,6-dimethyl-3-methoxy-para-phenylenediamine.

**21.** Composition according to Claim 20, characterised in that the compound of formula (I) is para-phenylenediamine.

**22.** Composition according to Claims 18 to 21, characterised in that the compounds of formula (II) are chosen from N,N'-bis(4-aminophenyl)tetramethylenediamine, N,N'-bis(β-diethylaminoethyl)-N,N'-bis(4-aminophenyl)tetramethylenediamine, N-(4-hydroxyphenyl)-N'-(4-aminophenyl)-ethylenediamine, N,N'-bis(β-hydroxyethyl)-N,N'-bis(4-aminophenyl)-ethylenediamine, N,N'-bis(4-aminophenyl)-1,3-diamino-2-propanol, N,N'-bis(β-hydroxyethyl)-N,N'-bis(4-aminophenyl)-1,3-diamino-2-propanol and N,N'-bis(ethyl)-N,N'-bis(4-amino-3-methylphenyl)ethylenediamine.

**23.** Process for dyeing keratinous fibres, especially human hair, characterised in that the following are applied on these fibres in a medium suitable for dyeing:

a) at least one dye precursor belonging to the family of single-unit bases (A) which are defined in Claim 1, and

b) at least one dye precursor belonging to the family of double-unit bases (B) corresponding to the formula (II) which are defined in Claim 1, in the presence of an oxidising agent; the mole ratio of the single-unit bases of the family (A) to the double-unit bases of the family (B) being between 3 and 10.

**24.** Process according to Claim 23, characterised in that the dye precursors of the family (A) and the dye precursors of the family (B) are applied in separate steps on the keratinous fibres.

**25.** Process according to Claim 23, characterised in that the dye precursors of the families (A) and (B) are applied on the keratinous fibres by means of a composition as defined in any one of Claims 18 to 22.

**26.** Process according to Claim 23, characterised in that the composition defined in any of Claims 18 to 22 is prepared immediately before use.

**27.** Multi-compartment device or kit for dyeing, characterised in that it comprises, in a first compartment, a composition containing, in a medium suitable for dyeing, at least one dye precursor of the family (A) of single-unit bases which are defined in Claim 1, and, in a second-compartment, a composition comprising, in a medium suitable for dyeing, at least one dye precursor of the family (B) of double-unit bases corresponding to the formula (II) which are defined in Claim 1; the mole ratio of the dye precursor of the family (A) to the dye precursor of the family (B) is between 3 and 10.